# EUROPEAN PATENT APPLICATION

(11) **EP 2 196 543 A1**
(43) Date of publication of application: **16.06.2010**
(21) Application number: 08171626.8
(22) Date of filing: 15.12.2008
(51) Int. Cl.: C12Q 1/68

(54) **Compositions and methods for micro-rna expression profiling of hepatocellular cancer**

(71) Applicant: Koninklijke Philips Electronics N.V., 5621 BA Eindhoven (NL)
(72) Inventor: The designation of the inventor has not yet been filed
(74) Representative: Jungen, Carmen

(57) **Abstract**

The present invention relates compositions and methods for microRNA (miRNA) expression profiling of hepatocellular cancer. In particular, the invention relates to a diagnostic kit of molecular markers for identifying one or more mammalian target cells exhibiting or having a predisposition to develop hepatocellular cancer, the kit comprising a plurality of nucleic acid molecules, each nucleic acid molecule encoding a microRNA sequence, wherein one or more of the plurality of nucleic acid molecules are differentially expressed in the target cells and in one or more control cells, and wherein the one or more differentially expressed nucleic acid molecules together represent a nucleic acid expression signature that is indicative for the presence of or the predisposition to develop hepatocellular cancer. The invention further relates to corresponding methods using such nucleic acid expression signatures for identifying one or more mammalian target cells exhibiting or having a predisposition to develop hepatocellular cancer as well as for preventing or treating such a condition. Finally, the invention is directed to a pharmaceutical composition for the prevention and/or treatment of hepatocellular cancer.

SEQUENCE LISTING

<110> Philips Intellectual Property & Standards
<120> Compositions and methods for micro-RNA expression profiling of
      hepatocellular cancer

<130> PH011746 EP1

<140> not assigned yet
<141> 2008-XX-XX

<160> 99

<170> PatentIn version 3.3


<210> 1
<211> 23
<212> RNA
<213> Homo sapiens

<220>
<221> misc_feature
<223> hsa-miR-221 microRNA

<400> 1
agcuacauug ucugcugggu uuc                                              23


<210> 2
<211> 23
<212> RNA
<213> Homo sapiens

<220>
<221> misc_feature
<223> hsa-miR-324-5p microRNA

<400> 2
   cgcauccccu agggcauugg ugu                                               23


<210> 3
<211> 23
<212> RNA
<213> Homo sapiens

<220>
<221> misc_feature
<223> hsa-miR-96 microRNA

<400> 3
uuuggcacua gcacauuuuu gcu                                              23


<210> 4
<211> 23
<212> RNA
<213> Homo sapiens

<220>
<221> misc_feature

<223> hsa-miR-18a microRNA

<400> 4
uaaggugcau cuagugcaga uag                                              23


<210> 5
<211> 23
<212> RNA
<213> Homo sapiens

<220>
<221> misc_feature
<223> hsa-miR-18b microRNA

<400> 5
uaaggugcau cuagugcagu uag                                              23


<210> 6
<211> 22
<212> RNA
<213> Homo sapiens

<220>
<221> misc_feature
<223> hsa-miR-30d microRNA

<400> 6
uguaaacauc cccgacugga ag                                               22


<210> 7
<211> 21
<212> RNA
<213> Homo sapiens

<220>
<221> misc_feature
<223> hsa-miR-331-3p microRNA

<400> 7
gccccugggc cuauccuaga a                                                21


<210> 8
<211> 22
<212> RNA
<213> Homo sapiens

<220>
<221> misc_feature
<223> hsa-miR-183 microRNA

<400> 8
uauggcacug guagaauuca cu                                               22


<210> 9
<211> 21
<212> RNA
<213> Homo sapiens

<220>
<221> misc_feature
<223> hsa-miR-551b microRNA

<400> 9
gcgacccaua cuugguuuca g                                                21


<210> 10
<211> 22
<212> RNA
<213> Homo sapiens

<220>
<221> misc_feature
<223> hsa-miR-139-5p microRNA

<400> 10
ucuacagugc acgugucucc ag                                               22


<210> 11
<211> 20
<212> RNA
<213> Homo sapiens

<220>
<221> misc_feature
<223> hsa-miR-144 microRNA

<400> 11
uacaguauag augauguacu                                                  20


<210> 12
<211> 22
<212> RNA
<213> Homo sapiens

<220>
<221> misc_feature
<223> hsa-miR-144* microRNA

<400> 12
ggauaucauc auauacugua ag                                               22


<210> 13
<211> 22
<212> RNA
<213> Homo sapiens

<220>
<221> misc_feature
<223> hsa-miR-450a microRNA

<400> 13
uuuugcgaug uguuccuaau au                                               22


<210> 14
<211> 22
<212> RNA

<213> Homo sapiens

<220>
<221> misc_feature
<223> hsa-miR-100 microRNA

<400> 14
aacccguaga uccgaacuug ug                                                22


<210> 15
<211> 21
<212> RNA
<213> Homo sapiens

<220>
<221> misc_feature
<223> hsa-miR-101 microRNA

<400> 15
uacaguacug ugauaacuga a                                                 21


<210> 16
<211> 23
<212> RNA
<213> Homo sapiens

<220>
<221> misc_feature
<223> hsa-miR-10a microRNA

<400> 16
uacccuguag auccgaauuu gug                                               23


<210> 17
<211> 22
<212> RNA
<213> Homo sapiens

<220>
<221> misc_feature
<223> hsa-miR-139-3p microRNA

<400> 17
ggagacgcgg cccuguugga gu                                                22


<210> 18
<211> 22
<212> RNA
<213> Homo sapiens

<220>
<221> misc_feature
<223> hsa-miR-148a microRNA

<400> 18
ucagugcacu acagaacuuu gu                                                22


<210> 19

<211> 23
<212> RNA
<213> Homo sapiens

<220>
<221> misc_feature
<223> hsa-miR-30c microRNA

<400> 19
uguaaacauc cuacacucuc agc                                              23


<210> 20
<211> 22
<212> RNA
<213> Homo sapiens

<220>
<221> misc_feature
<223> hsa-miR-122* microRNA

<400> 20
aacgccauua ucacacuaaa ua                                               22


<210> 21
<211> 21
<212> RNA
<213> Homo sapiens

<220>
<221> misc_feature
<223> hsa-miR-152 microRNA

<400> 21
ucagugcaug acagaacuug g                                                21


<210> 22
<211> 22
<212> RNA
<213> Homo sapiens

<220>
<221> misc_feature
<223> hsa-miR-24-1* microRNA

<400> 22
ugccuacuga gcugauauca gu                                               22


<210> 23
<211> 21
<212> RNA
<213> Homo sapiens

<220>
<221> misc_feature
<223> hsa-miR-26b microRNA

<400> 23
uucaaguaau ucaggauagg u                                                21

<210> 24
<211> 22
<212> RNA
<213> Homo sapiens

<220>
<221> misc_feature
<223> hsa-miR-29c* microRNA

<400> 24
ugaccgauuu cuccuggugu uc                                                22


<210> 25
<211> 22
<212> RNA
<213> Homo sapiens

<220>
<221> misc_feature
<223> hsa-miR-30e microRNA

<400> 25
uguaaacauc cuugacugga ag                                                22


<210> 26
<211> 22
<212> RNA
<213> Homo sapiens

<220>
<221> misc_feature
<223> hsa-miR-30e* microRNA

<400> 26
cuuucagucg gauguuuaca gc                                                22


<210> 27
<211> 21
<212> RNA
<213> Homo sapiens

<220>
<221> misc_feature
<223> hsa-miR-378 microRNA

<400> 27
acuggacuug gagucagaag g                                                 21


<210> 28
<211> 22
<212> RNA
<213> Homo sapiens

<220>
<221> misc_feature
<223> hsa-miR-378* microRNA

<400> 28

cuccugacuc cagguccugu gu                                              22

<210> 29
<211> 22
<212> RNA
<213> Homo sapiens

<220>
<221> misc_feature
<223> hsa-miR-424 microRNA

<400> 29
cagcagcaau ucauguuuug aa                                              22

<210> 30
<211> 21
<212> RNA
<213> Homo sapiens

<220>
<221> misc_feature
<223> hsa-miR-455-3p microRNA

<400> 30
gcaguccaug ggcauauaca c                                               21

<210> 31
<211> 22
<212> RNA
<213> Homo sapiens

<220>
<221> misc_feature
<223> hsa-miR-455-5p microRNA

<400> 31
uaugugccuu uggacuacau cg                                              22

<210> 32
<211> 22
<212> RNA
<213> Homo sapiens

<220>
<221> misc_feature
<223> hsa-miR-505 microRNA

<400> 32
cgucaacacu ugcugguuuc cu                                              22

<210> 33
<211> 22
<212> RNA
<213> Homo sapiens

<220>
<221> misc_feature
<223> hsa-miR-542-3p microRNA

<400> 33
ugugacagau ugauaacuga aa                                              22


<210> 34
<211> 22
<212> RNA
<213> Homo sapiens

<220>
<221> misc_feature
<223> hsa-miR-99a microRNA

<400> 34
aacccguaga uccgaucuug ug                                              22


<210> 35
<211> 22
<212> RNA
<213> Homo sapiens

<220>
<221> misc_feature
<223> hsa-miR-29c microRNA

<400> 35
uagcaccauu ugaaaucggu ua                                              22


<210> 36
<211> 22
<212> RNA
<213> Homo sapiens

<220>
<221> misc_feature
<223> hsa-miR-338-3p microRNA

<400> 36
uccagcauca gugauuuugu ug                                              22


<210> 37
<211> 21
<212> RNA
<213> Homo sapiens

<220>
<221> misc_feature
<223> hsa-miR-224 microRNA

<400> 37
caagucacua gugguuccgu u                                               21


<210> 38
<211> 22
<212> RNA
<213> Homo sapiens

<220>

<221> misc_feature
<223> hsa-miR-532-3p microRNA

<400> 38
ccucccacac ccaaggcuug ca                                                22


<210> 39
<211> 22
<212> RNA
<213> Homo sapiens

<220>
<221> misc_feature
<223> hsa-miR-663 microRNA

<400> 39
aggcggggcg ccgcgggacc gc                                                22


<210> 40
<211> 22
<212> RNA
<213> Homo sapiens

<220>
<221> misc_feature
<223> hsa-miR-99b microRNA

<400> 40
cacccguaga accgaccuug cg                                                22


<210> 41
<211> 24
<212> RNA
<213> Homo sapiens

<220>
<221> misc_feature
<223> hsa-miR-362-5p microRNA

<400> 41
aauccuugga accuaggugu gagu                                              24


<210> 42
<211> 21
<212> RNA
<213> Homo sapiens

<220>
<221> misc_feature
<223> hsa-miR-652 microRNA

<400> 42
aauggcgcca cuaggguugu g                                                 21


<210> 43
<211> 21
<212> RNA
<213> Homo sapiens

<220>
<221> misc_feature
<223> hsa-miR-222 microRNA

<400> 43
agcuacaucu ggcuacuggg u                                                21


<210> 44
<211> 22
<212> RNA
<213> Homo sapiens

<220>
<221> misc_feature
<223> hsa-miR-501-3p microRNA

<400> 44
aaugcacccg ggcaaggauu cu                                               22


<210> 45
<211> 22
<212> RNA
<213> Homo sapiens

<220>
<221> misc_feature
<223> hsa-miR-375 microRNA

<400> 45
uuuguucguu cggcucgcgu ga                                               22


<210> 46
<211> 22
<212> RNA
<213> Homo sapiens

<220>
<221> misc_feature
<223> hsa-miR-451 microRNA

<400> 46
aaaccguuac cauuacugag uu                                               22


<210> 47
<211> 23
<212> RNA
<213> Homo sapiens

<220>
<221> misc_feature
<223> hsa-miR-335 microRNA

<400> 47
ucaagagcaa uaacgaaaaa ugu                                              23


<210> 48
<211> 22

<212> RNA
<213> Homo sapiens

<220>
<221> misc_feature
<223> hsa-miR-130b microRNA

<400> 48
cagugcaaug augaaagggc au                                                22


<210> 49
<211> 22
<212> RNA
<213> Homo sapiens

<220>
<221> misc_feature
<223> hsa-miR-132 microRNA

<400> 49
uaacagucua cagccauggu cg                                                22


<210> 50
<211> 21
<212> RNA
<213> Homo sapiens

<220>
<221> misc_feature
<223> hsa-miR-106b microRNA

<400> 50
uaaagugcug acagugcaga u                                                 21


<210> 51
<211> 24
<212> RNA
<213> Homo sapiens

<220>
<221> misc_feature
<223> hsa-miR-125a-5p microRNA

<400> 51
ucccugagac ccuuuaaccu guga                                              24


<210> 52
<211> 21
<212> RNA
<213> Homo sapiens

<220>
<221> misc_feature
<223> hsa-miR-151-3p microRNA

<400> 52
cuagacugaa gcuccuugag g                                                 21

<210> 53
<211> 23
<212> RNA
<213> Homo sapiens

<220>
<221> misc_feature
<223> hsa-miR-17 microRNA

<400> 53
caaagugcuu acagugcagg uag                                              23


<210> 54
<211> 21
<212> RNA
<213> Homo sapiens

<220>
<221> misc_feature
<223> hsa-miR-21* microRNA

<400> 54
caacaccagu cgaugggcug u                                                21


<210> 55
<211> XX
<212> RNA
<213> Homo sapiens

<220>
<221> misc_feature
<223> hsa-miR-25 microRNA

<400> 55
cauugcacuu gucucggucu ga                                               22


<210> 56
<211> 23
<212> RNA
<213> Homo sapiens

<220>
<221> misc_feature
<223> hsa-miR-301a microRNA

<400> 56
cagugcaaua guauugucaa agc                                              23


<210> 57
<211> 23
<212> RNA
<213> Homo sapiens

<220>
<221> misc_feature
<223> hsa-miR-423-5p microRNA

<400> 57
ugaggggcag agagcgagac uuu                                              23

<210> 58
<211> 23
<212> RNA
<213> Homo sapiens

<220>
<221> misc_feature
<223> hsa-miR-425 microRNA

<400> 58
aaugacacga ucacucccgu uga                                              23


<210> 59
<211> 22
<212> RNA
<213> Homo sapiens

<220>
<221> misc_feature
<223> hsa-miR-484 microRNA

<400> 59
ucaggcucag uccccucccg au                                               22


<210> 60
<211> 22
<212> RNA
<213> Homo sapiens

<220>
<221> misc_feature
<223> hsa-miR-500* microRNA

<400> 60
augcaccugg gcaaggauuc ug                                               22


<210> 61
<211> 22
<212> RNA
<213> Homo sapiens

<220>
<221> misc_feature
<223> hsa-miR-502-3p microRNA

<400> 61
aaugcaccug ggcaaggauu ca                                               22


<210> 62
<211> 21
<212> RNA
<213> Homo sapiens

<220>
<221> misc_feature
<223> hsa-miR-502-5p microRNA

<400> 62
auccuugcua ucugggugcu a                                                21

<210> 63
<211> 22
<212> RNA
<213> Homo sapiens

<220>
<221> misc_feature
<223> hsa-miR-532-5p microRNA

<400> 63
caugccuuga guguaggacc gu                                               22

<210> 64
<211> 22
<212> RNA
<213> Homo sapiens

<220>
<221> misc_feature
<223> hsa-miR-769-5p microRNA

<400> 64
ugagaccucu ggguucugag cu                                               22

<210> 65
<211> 23
<212> RNA
<213> Homo sapiens

<220>
<221> misc_feature
<223> hsa-miR-93 microRNA

<400> 65
caaagugcug uucgugcagg uag                                              23

<210> 66
<211> 23
<212> RNA
<213> Homo sapiens

<220>
<221> misc_feature
<223> hsa-miR-181b microRNA

<400> 66
aacauucauu gcugucggug ggu                                              23

<210> 67
<211> 22
<212> RNA
<213> Homo sapiens

<220>
<221> misc_feature

<223> hsa-miR-501-5p microRNA

<400> 67
   aauccuuugu cccuggguga ga                                              22


<210> 68
<211> 22
<212> RNA
<213> Homo sapiens

<220>
<221> misc_feature
<223> hsa-miR-194 microRNA

<400> 68
uguaacagca acuccaugug ga                                              22


<210> 69
<211> 22
<212> RNA
<213> Homo sapiens

<220>
<221> misc_feature
<223> hsa-miR-199b-3p microRNA

<400> 69
acaguagucu gcacauuggu ua                                              22


<210> 70
<211> 21
<212> RNA
<213> Homo sapiens

<220>
<221> misc_feature
<223> hsa-miR-215 microRNA

<400> 70
augaccuaug aauugacaga c                                               21


<210> 71
<211> 23
<212> RNA
<213> Homo sapiens

<220>
<221> misc_feature
<223> hsa-miR-199b-5p microRNA

<400> 71
cccaguguuu agacuaucug uuc                                             23


<210> 72
<211> 22
<212> RNA
<213> Homo sapiens

<220>
<221> misc_feature
<223> hsa-miR-21 microRNA

<400> 72
uagcuuauca gacugauguu ga                                                22


<210> 73
<211> 23
<212> RNA
<213> Homo sapiens

<220>
<221> misc_feature
<223> hsa-miR-19b microRNA

<400> 73
ugugcaaauc caugcaaaac uga                                               23


<210> 74
<211> 23
<212> RNA
<213> Homo sapiens

<220>
<221> misc_feature
<223> hsa-miR-103 microRNA

<400> 74
agcagcauug uacagggcua uga                                               23


<210> 75
<211> 21
<212> RNA
<213> Homo sapiens

<220>
<221> misc_feature
<223> hsa-miR-23a microRNA

<400> 75
aucacauugc cagggauuuc c                                                 21


<210> 76
<211> 22
<212> RNA
<213> Homo sapiens

<220>
<221> misc_feature
<223> hsa-miR-125b-2* microRNA

<400> 76
ucacaaguca ggcucuuggg ac                                                22


<210> 77
<211> 22
<212> RNA

<213> Homo sapiens

<220>
<221> misc_feature
<223> hsa-miR-30c-2* microRNA

<400> 77
cugggagaag gcuguuuacu cu                                                22


<210> 78
<211> 22
<212> RNA
<213> Homo sapiens

<220>
<221> misc_feature
<223> hsa-miR-505* microRNA

<400> 78
gggagccagg aaguauugau gu                                                22


<210> 79
<211> 22
<212> RNA
<213> Homo sapiens

<220>
<221> misc_feature
<223> hsa-miR-203 microRNA

<400> 79
gugaaauguu uaggaccacu ag                                                22


<210> 80
<211> 22
<212> RNA
<213> Homo sapiens

<220>
<221> misc_feature
<223> hsa-let-7c microRNA

<400> 80
ugagguagua gguuguaugg uu                                                22


<210> 81
<211> 22
<212> RNA
<213> Homo sapiens

<220>
<221> misc_feature
<223> hsa-miR-122 microRNA

<400> 81
uggaguguga caaugguguu ug                                                22


<210> 82

<211> 22
<212> RNA
<213> Homo sapiens

<220>
<221> misc_feature
<223> hsa-miR-125b microRNA

<400> 82
ucccugagac ccuaacuugu ga                                                22


<210> 83
<211> 22
<212> RNA
<213> Homo sapiens

<220>
<221> misc_feature
<223> hsa-miR-130a microRNA

<400> 83
cagugcaaug uuaaaagggc au                                                22


<210> 84
<211> XX
<212> RNA
<213> Homo sapiens

<220>
<221> misc_feature
<223> hsa-miR-195 microRNA

<400> 84
XXXXXXXXXX XXXXXXXXXX                                                   XX


<210> 85
<211> 21
<212> RNA
<213> Homo sapiens

<220>
<221> misc_feature
<223> hsa-miR-30a microRNA

<400> 85
uagcagcaca gaaauauugg c                                                 21


<210> 86
<211> 21
<212> RNA
<213> Homo sapiens

<220>
<221> misc_feature
<223> hsa-miR-126* microRNA

<400> 86
cauuauuacu uuugguacgc g                                                 21

<210> 87
<211> 21
<212> RNA
<213> Homo sapiens

<220>
<221> misc_feature
<223> hsa-miR-128 microRNA

<400> 87
ucacagugaa ccggucucuu u                                                21


<210> 88
<211> 22
<212> RNA
<213> Homo sapiens

<220>
<221> misc_feature
<223> hsa-miR-192* microRNA

<400> 88
cugccaauuc cauaggucac ag                                               22


<210> 89
<211> 22
<212> RNA
<213> Homo sapiens

<220>
<221> misc_feature
<223> hsa-miR-28-5p microRNA

<400> 89
aaggagcuca cagucuauug ag                                               22


<210> 90
<211> 22
<212> RNA
<213> Homo sapiens

<220>
<221> misc_feature
<223> hsa-miR-30a* microRNA

<400> 90
cuuucagucg gauguuugca gc                                               22


<210> 91
<211> 22
<212> RNA
<213> Homo sapiens

<220>
<221> misc_feature
<223> hsa-miR-30b* microRNA

<400> 91

cugggaggug gauguuuacu uc                                              22

<210> 92
<211> 22
<212> RNA
<213> Homo sapiens

<220>
<221> misc_feature
<223> hsa-miR-486-5p microRNA

<400> 92
uccuguacug agcugccccg ag                                              22

<210> 93
<211> 21
<212> RNA
<213> Homo sapiens

<220>
<221> misc_feature
<223> hsa-miR-497 microRNA

<400> 93
cagcagcaca cugugguuug u                                               21

<210> 94
<211> 23
<212> RNA
<213> Homo sapiens

<220>
<221> misc_feature
<223> hsa-miR-542-5p microRNA

<400> 94
ucggggauca ucaugucacg aga                                             23

<210> 95
<211> 19
<212> RNA
<213> Homo sapiens

<220>
<221> misc_feature
<223> hsa-miR-564 microRNA

<400> 95
aggcacggug ucagcaggc                                                  19

<210> 96
<211> 22
<212> RNA
<213> Homo sapiens

<220>
<221> misc_feature
<223> hsa-miR-590-5p microRNA

<400> 96
gagcuuauuc auaaaagugc ag                                                22

<210> 97
<211> 22
<212> RNA
<213> Homo sapiens

<220>
<221> misc_feature
<223> hsa-miR-340 microRNA

<400> 97
uuauaaagca augagacuga uu                                                22

<210> 98
<211> 23
<212> RNA
<213> Homo sapiens

<220>
<221> misc_feature
<223> hsa-miR-582-5p microRNA

<400> 98
uuacaguugu ucaaccaguu acu                                               23

<210> 99
<211> 22
<212> RNA
<213> Homo sapiens

<220>
<221> misc_feature
<223> hsa-miR-194* microRNA

<400> 99
ccaguggggc ugcuguuauc ug                                                22

## Description

### FIELD OF THE INVENTION

The present invention relates to compositions and methods for microRNA expression profiling of hepatocellular cancer.

### BACKGROUND OF THE INVENTION

Hepatocellular cancer (also referred to as hepatocellular carcinoma, HCC) is one of the most common solid malignancies worldwide. It represents the major histological type of liver cancer and likely accounts for 70%-85% of all cases of liver cancer. About 500.000 new cases occur every year worldwide, with almost the same number of fatalities, reflecting a lack of effective early detection and treatment options (reviewed, e.g., in Thorgeirsson, S.S. and Grisham, J.W. (2002) Nat. Genet. 31, 339-346; Parkin, D.M. et al. (2005) CA Cancer J. Clin. 55, 74-108).

Hepatocellular cancer thus represents a type of an extremely poor prognostic cancer. The dismal outcome has been attributed to the major hallmarks of hepatocellular cancer, namely intra-hepatic metastases or postsurgical recurrence. New tumor colonies frequently invade into the major branches of the portal vein and possibly other parts of the liver (reviewed, for example, in Tang, Z.Y. (2001) World J Gastroenterol 7, 445-454; Chambers, A.F. et al. (2002) Nat. Rev. Cancer 2, 563-572). Resection and/or liver transplantation are the best options for a potential cure. However, only about 10%-20% of patients with hepatocellular cancer, defined by parameters of relatively normal liver function and a manageable tumor lesion as determined by the available clinical staging systems, are currently eligible for surgical intervention. Moreover, patients who were resected often have a high frequency of metastasis/recurrence, and postoperative 5-year survival is only 30%-40%.

Hepatocellular tumors comprise diverse benign and malignant neoplasms. Although the phenotype can be broadly distinguished histologically, these tumours can vary widely in their clinical behavior and prognosis. Therefore, early detection of such tumors would be desirable in order to discriminate these different types of tumor and to enable a reliably risk assessment of patients exhibiting or being supposed to have a (benign) pre-cancerous state whether or not the pre-cancerous state will progress in a carcinoma. The prediction of cancer progression may be used to guide the therapy decision in patients exhibiting a type of hepatocellular cancer and thus can markedly help to improve long-term survival.

Although early detection and surgical removal of hepatocellular tumors have significantly improved the patient survival in recent years, the majority of tumors is still not detected early in tumor progression, that is, in a non-fatal stage. Currently, only one serum marker (alpha-fetoprotein; AFP) is generally used for the early detection of hepatocellular cancer (cf., e.g., Mizejewski, G.J. (2003) Expert Rev. Anticancer Ther. 2, 709-735; Paul, S.B. et al. (2007) Oncology 72, Suppl. 1, 117-123). However, this marker has a low specificity and is frequently inadequate because of false-positive results. The serum AFP test can readily detect hepatocellular tumors in only 60% of the patients. On the other hand, in a large number of cirrhosis patients, AFP can be elevated in the absence of cancerous states.

Therefore, the discovery of new biomarkers would be of utmost clinical importance, particularly if these markers enable a diagnosis at an early stage of tumor progression in order to allow early stage treatment of carcinomas while avoiding unnecessary surgical intervention. Ideally, such markers should enable the identification of a carcinoma at a stage where the presence of malignant cells is not yet detectable by *in situ* techniques or microscopic analysis of biopsy or resection material.

Many diagnostic assays are also hampered by the fact that they are typically based on the analysis of only a single molecular marker, which might affect detection reliability and/or accuracy. In addition, a single marker normally does not enable detailed predictions concerning latency stages, tumor progression, and the like. Thus, there is still a continuing need for the identification of alternative molecular markers and assay formats overcoming these limitations.

One approach to address this issue might be based on small regulatory RNA molecules, in particular on microRNAs (miRNAs) which, constitute an evolutionary conserved class of endogenously expressed small non-coding RNAs of 20-25 nucleotides (nt) in size that can mediate the expression of target mRNAs and thus - since their discovery about ten years ago - have been implicated with critical functions in cellular development, differentiation, proliferation, and apoptosis.

MiRNAs are produced from primary transcripts that are processed to stem-loop structured precursors (pre-miRNAs) by the RNase III Drosha. After transport to the cytoplasm, another RNase III termed Dicer cleaves of the loop of the pre-miRNA hairpin to form a short double-stranded (ds) RNA, one strand of which is incorporated as mature miRNA into a miRNA-protein (miRNP). The miRNA guides the miRNPs to their target mRNAs where they exert their function (reviewed, e.g. in Bartel, D.P. (2004) Cell 23, 281-292; He, L. and Hannon, G.J. (2004) Nat. Rev. Genet. 5, 522-531).

Depending on the degree of complementarity between the miRNA and its target, miRNAs can guide different regulatory processes. Target mRNAs that are highly complementary to miRNAs are specifically cleaved by mechanisms identical to RNA interference (RNAi). Thus, in such scenario, the miRNAs function as short interfering RNAs (siRNAs). Target mRNAs with less complementarity to miRNAs are either directed to cellular degradation pathways or are translationally repressed without affecting the mRNA level. However, the mechanism of how miRNAs repress translation of their target mRNAs is still a matter of controversy.

Emerging data available indicate that dysregulation of miRNA expression may *inter alia* be associated with the development and/or progression of certain types of cancer. For example, two miRNAs, *miR-15* and *miR-16-1*, were shown to map to a genetic locus that is deleted in chronic lymphatic leukemia (CLL) and it was found that in about 70% of the CLL patients, both miRNA genes are deleted or down-regulated. Furthermore, down-regulation of *miR-143* and *miR-145* was observed in colorectal neoplasia, whereas expression of the miRNA *let-7* is frequently reduced in lung cancers (Michael, M.Z. et al. (2003) Mol. Cancer Res. 1, 882-891; Mayr, C. et al. (2007) Science 315, 1576-1579). In fact, it has been speculated based on cancer-associated alterations in miRNA expression and the observation that miRNAs are frequently located at genomic regions involved in cancers that miRNAs may act both as tumor suppressors and as oncogenes (reviewed, e.g., in Esquela-Kerscher, A. and Slack, F.J (2006) Nat. Rev. Cancer 6, 259-269; Calin, G.A. and Croce, C.M. (2007) J. Clin. Invest. 117, 2059-2066; Blenkiron, C. and Miska, E.A. (2007) Hum. Mol. Genet. 16, R106-R113).

More systematic bead-based flow cytometric miRNA expression analyses have revealed a global miRNAs regulation in tumors indicating that miRNA profiling of host cells might indeed be suitable for cancer diagnosis (cf., e.g., Lu J. et al. (2005) Nature 435, 834-838; Volinia, S. et al. (2006) Proc. Natl. Acad. Sci. USA 103, 2257-2261) and various miRNAs whose expression appears characteristic for a particular tumor have been identified (Calin, G.A. and Croce, C.M. (2007), *supra*). However, to date only few of these aberrantly expressed miRNAs have been directly linked with clinically relevant prognostic factors for tumor development and/or progression.

Several studies have reported miRNA expression profiling in human hepatocellular cancer (cf., e.g., Murakami, Y. et al. (2006) Oncogene 25, 2537-2545; Li, W. et al. (2008) Int. J. Cancer 123, 1616-1622; Huang, Y.S. et al. (2008) Hepatology 23, 87-94; Ladeiro, Y. et al. (2008) Hepatology 47, 1955-1963; Jiang, J. et al. (2008) Clin. Cancer Res. 14, 419-427). Consistently, these studies have shown that specific miRNAs are aberrantly expressed in malignant cells or tissues as compared to nonmalignant hepatocytes or tissue. Thus, such miRNAs may provide insights into cellular processes involved in malignant transformation. However, these studies focused on tumor tissues without separating the malignant cells. Therefore, 40% of the reported HCC-associated miRNAs showed different (partially even opposing) regulatory patterns across the different studies. Furthermore, no data are available on a correlation between miRNA expression and early stages of HCC.

Thus, there still remains a need for (a set of) diagnostic markers, particularly in form of a "expression signature" or a "molecular footprint", that enable the rapid, reliable and cost-saving identification and/or treatment of cells exhibiting or having a predisposition to develop hepatocellular cancer. In addition, there is also a continuing need for corresponding methods both for the identification and for the treatment of target cells displaying such a cancerous phenotype.

### OBJECT AND SUMMARY OF THE INVENTION

It is an objective of the present invention to provide novel approaches for diagnosing and/or treating hepatocellular cancer and/or the predisposition for developing such a condition by determining a plurality of nucleic acid molecules, each nucleic acid molecule encoding a microRNA (miRNA) sequence, wherein one or more of the plurality of nucleic acid molecules are differentially expressed in the target cells analyzed as compared to healthy control cells, and wherein the one or more differentially expressed nucleic acid molecules together represent a nucleic acid expression signature that is indicative for the presence of or the predisposition to develop hepatocellular cancer.

More specifically, it is an object of the invention to provide compositions for discriminating different types of hepatocellular cancer and/or different stages of tumor progression.

Furthermore, it is an object of the invention to provide corresponding methods for identifying one or more mammalian target cells exhibiting or having a predisposition to develop hepatocellular cancer as well as for preventing or treating such a condition.

These objectives as well as others, which will become apparent from the ensuing description, are attained by the subject matter of the independent claims. Some of the preferred embodiments of the present invention are defined by the subject matter of the dependent claims.

In a first aspect, the present invention relates to a diagnostic kit of molecular markers for identifying one or more mammalian target cells exhibiting or having a predisposition to develop hepatocellular cancer, the kit comprising a plurality of nucleic acid molecules, each nucleic acid molecule encoding a microRNA sequence, wherein one or more of the plurality of nucleic acid molecules are differentially expressed in the target cells and in one or more control cells, and wherein the one or more differentially expressed nucleic acid molecules together represent a nucleic acid expression signature that is indicative for the presence of or the predisposition to develop hepatocellular cancer.

The nucleic acid expression signature, as defined herein, may comprise at least five nucleic acid molecules, preferably at least eight nucleic acid molecules, and particularly preferably at least twelve nucleic acid molecules.

In specific embodiments, the nucleic acid expression signature comprises at least one nucleic acid molecule encoding a microRNA sequence whose expression is up-regulated in the one or more target cells compared to the one or more control cells and at least one nucleic acid molecule encoding a microRNA sequence whose expression is down-regulated in the one or more target cells compared to the one or more control cells.

In preferred embodiments, the nucleic acid expression signature comprises any one or more of the nucleic acid molecules encoding hsa-mir-221, hsa-mir-324-5p, hsa-mir-96, hsa-mir-18a, hsa-mir-18b, hsa-mir-30d, hsa-mir-331-3p, hsa-mir-183, hsa-mir-551b, hsa-mir-139-5p, hsa-mir-144, hsa-mir-144*, and hsa-mir-450a.

Particularly preferably, the expression of any one or more of the nucleic acid molecules encoding hsa-mir-221, hsa-mir-324-5p, hsa-mir-96, hsa-mir-18a, hsa-mir-18b, hsa-mir-30d, hsa-mir-331-3p, hsa-mir-183, and hsa-mir-551b is up-regulated and the expression of any one or more of the nucleic acid molecules hsa-mir-139-5p, hsa-mir-144, hsa-mir-144*, and hsa-mir-450a is down-regulated in the in the one or more target cells compared to the one or more control cells.

In further specific embodiments of the invention, the diagnostic kit is for the further use of discriminating hepatocellular cancer selected from the group consisting of hepatocellular carcinoma and carcinoma embolus.

In preferred embodiments, the hepatocellular cancer is hepatocellular carcinoma, and the nucleic acid expression signature comprises any one or more of the nucleic acid molecules encoding hsa-miR-224, hsa-miR-532-3p, hsa-miR-663, hsa-miR-99b, hsa-miR-362-5p, hsa-miR-652, hsa-miR-222, hsa-miR-501-3p, hsa-miR-375, hsa-miR-451, and hsa-miR-335.

Particularly preferably, the expression of any one or more of the nucleic acid molecules encoding hsa-miR-224, hsa-miR-532-3p, hsa-miR-663, hsa-miR-99b, hsa-miR-362-5p, hsa-miR-652, hsa-miR-222, and hsa-miR-501-3p is up-regulated and the expression of any one or more of the nucleic acid molecules hsa-miR-375, hsa-miR-451, and hsa-miR-335 is down-regulated in the in the one or more target cells compared to the one or more control cells

In other preferred embodiments, the hepatocellular cancer is carcinoma embolus, and the nucleic acid expression signature comprises any one or more of the nucleic acid molecules encoding hsa-miR-199b-Sp, hsa-miR-21, hsa-miR-19b, hsa-miR-103, hsa-miR-23a, hsa-miR-125b-2*, hsa-miR-30c-2*, hsa-miR-505*, and hsa-miR-203.

Particularly preferably, the expression of any one or more of the nucleic acid molecules encoding hsa-miR-199b-5p, hsa-miR-21, hsa-miR-19b, hsa-miR-103, and hsa-miR-23a is up-regulated and the expression of any one or more of the nucleic acid molecules hsa-miR-125b-2*, hsa-miR-30c-2*, hsa-miR-505*, and hsa-miR-203 is down-regulated in the in the one or more target cells compared to the one or more control cells.

In a second aspect, the present invention relates to a method for identifying one or more mammalian target cells exhibiting or having a predisposition to develop hepatocellular cancer, the method comprising: (a) determining in the one or more target cells the expression levels of a plurality of nucleic acid molecules, each nucleic acid molecule encoding a microRNA sequence; (b) determining the expression levels of the plurality of nucleic acid molecules in one or more control cells; and (c) identifying from the plurality of nucleic acid molecules one or more nucleic acid molecules that are differentially expressed in the target and control cells by comparing the respective expression levels obtained in steps (a) and (b), wherein the one or more differentially expressed nucleic acid molecules together represent a nucleic acid expression signature, as defined herein, that is indicative for the presence of or the predisposition to develop hepatocellular cancer.

In preferred embodiments of the invention, the method is for the further use of discriminating hepatocellular cancer selected from the group consisting of hepatocellular carcinoma and carcinoma embolus.

In a third aspect, the present invention relates to a method for preventing or treating hepatocellular cancer in one or more mammalian target cells, the method comprising: (a) identifying in one or more target cells a nucleic acid expression signature by using a method, as defined herein; and (b) modifying in the one or more cells the expression of one or more nucleic acid molecules encoding a microRNA sequence that is/are comprised in the nucleic acid expression signature in such way that the expression of a nucleic acid molecule whose expression is up-regulated in the one or more target cells is down-regulated and the expression of a nucleic acid molecule whose expression is down-regulated in the one or more target cells is up-regulated.

In a forth aspect, the present invention relates to a pharmaceutical composition for the prevention and/or treatment of hepatocellular cancer in one or more mammalian target cells, the composition comprising one or more nucleic acid molecules, each nucleic acid molecule encoding a sequence that is at least partially complementary to a microRNA sequence encoded by a nucleic acid molecule whose expression is up-regulated in the one or more target cells, as defined herein, and/or that corresponds to a microRNA sequence encoded by a nucleic acid molecule whose expression is down-regulated in the one or more target cells, as defined herein.

Finally, in a fifth aspect, the present invention relates to the use of said pharmaceutical composition for the manufacture of a medicament for the prevention and/or treatment of hepatocellular cancer.

Other embodiments of the present invention will become apparent from the detailed description hereinafter.

### BRIEF DESCRIPTION OF THE DRAWINGS

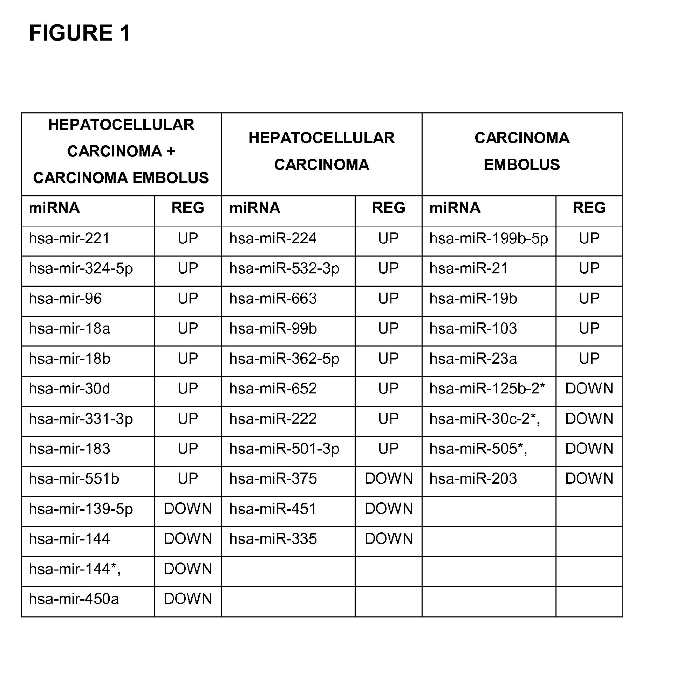

- Figure 1: illustrates the human miRNAs comprised in particularly preferred expression signatures according to the present invention for identifying one or more target cells exhibiting or having a predisposition to develop hepatocellular cancer (i.e. hepatocellular carcinoma and carcinoma embolus) as well as for discriminating between hepatocellular carcinoma and carcinoma embolus, respectively. Also indicated are the expression levels ("REG" for "regulation") of these miRNAs in cancerous tissue as compared to (healthy) control tissue (i.e. an up-regulation or a down-regulation).

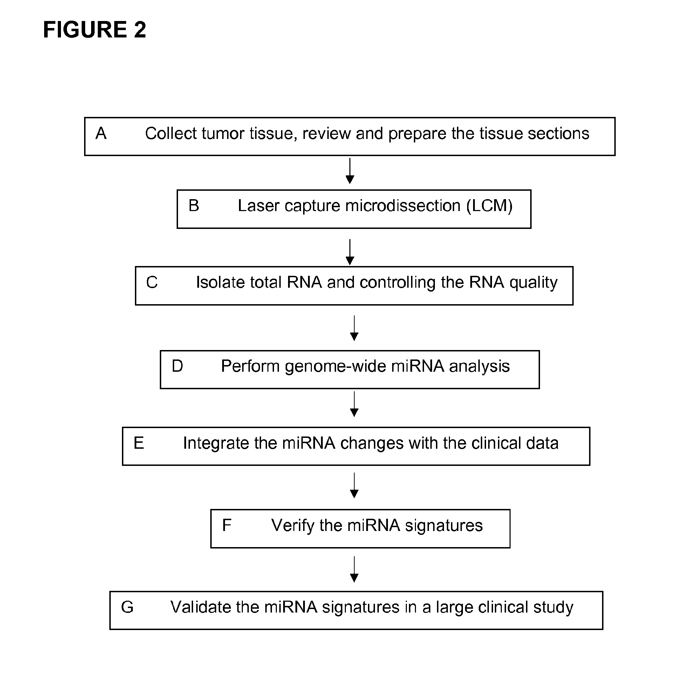

- Figure 2: depicts a flow chart schematically illustrating the essential method steps for determining an expression signature according to the present invention for identifying one or more target cells exhibiting or having a predisposition to develop hepatocellular cancer.

### DETAILED DESCRIPTION OF THE INVENTION

The present invention is based on the unexpected finding that cells exhibiting or having a predisposition to develop hepatocellular cancer (HCC) can be reliably identified and different types of HCC discriminated based on particular miRNA expression signatures both with high accuracy and sensitivity, wherein the expression signatures as defined herein typically comprises both up- and down-regulated human miRNAs. More specifically, said miRNA expression signatures - by analyzing the overall miRNA expression pattern and/or the respective individual miRNA expression level(s) - allow the detection of HCC at an early disease state as well as an evaluation of the risk that a benign pre-cancerous state transforms into a malignant carcinoma.

The present invention illustratively described in the following may suitably be practiced in the absence of any element or elements, limitation or limitations, not specifically disclosed herein.

The present invention will be described with respect to particular embodiments and with reference to certain drawings but the invention is not limited thereto but only by the claims. The drawings described are only schematic and are to be considered non-limiting.

Where the term "comprising" is used in the present description and claims, it does not exclude other elements or steps. For the purposes of the present invention, the term "consisting of" is considered to be a preferred embodiment of the term "comprising of". If hereinafter a group is defined to comprise at least a certain number of embodiments, this is also to be understood to disclose a group, which preferably consists only of these embodiments.

Where an indefinite or definite article is used when referring to a singular noun e.g. "a" or "an", "the", this includes a plural of that noun unless something else is specifically stated.

The term "about" in the context of the present invention denotes an interval of accuracy that the person skilled in the art will understand to still ensure the technical effect of the feature in question. The term typically indicates deviation from the indicated numerical value of ± 10%, and preferably ± 5%.

Furthermore, the terms first, second, third, (a), (b), (c), and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Further definitions of term will be given in the following in the context of which the terms are used.

The following terms or definitions are provided solely to aid in the understanding of the invention. These definitions should not be construed to have a scope less than understood by a person of ordinary skill in the art.

In a first aspect, the present invention relates to a diagnostic kit of molecular markers for identifying one or more mammalian target cells exhibiting or having a predisposition to develop hepatocellular cancer, the kit comprising a plurality of nucleic acid molecules, each nucleic acid molecule encoding a microRNA sequence, wherein one or more of the plurality of nucleic acid molecules are differentially expressed in the target cells and in one or more control cells, and wherein the one or more differentially expressed nucleic acid molecules together represent a nucleic acid expression signature that is indicative for the presence of or the predisposition to develop hepatocellular cancer.

The term "cancer" (also referred to as "carcinoma"), as used herein, generally denotes any type of malignant neoplasm, that is, any morphological and/or physiological alterations (based on genetic re-programming) of target cells exhibiting or having a predisposition to develop characteristics of a carcinoma as compared to unaffected (healthy) wild-type control cells. Examples of such alterations may relate *inter alia* to cell size and shape (enlargement or reduction), cell proliferation (increase in cell number), cell differentiation (change in physiological state), apoptosis (programmed cell death) or cell survival.

The term "hepatocellular" (or "hepatic"), as used herein, relates to the liver. Hence, the term "hepatocellular cancer" refers to cancerous growths in the liver.

The term "having a predisposition to develop cancer", as used herein, denotes any cellular phenotype being indicative for a pre-cancerous state, i.e. an intermediate state in the transformation of a normal cell into a tumor cell. In other words, the term denotes a state of risk of developing cancer.

The most common type of hepatocellular (liver) cancer is hepatocellular carcinoma (also referred to as "hepatoma" and commonly abbreviated as "HCC"). The term "hepatocellular carcinoma", as used herein, denotes a primary malignancy of the liver. Most cases of HCC are secondary to either a viral hepatitide infection (hepatitis B or C) or cirrhosis (alcoholism being the most common cause of hepatic cirrhosis). In countries where hepatitis is not endemic, most malignant cancers in the liver are not primary HCC but metastasis (spread) of cancer from elsewhere in the body, e.g. the colon. Treatment options of HCC and prognosis are dependent on many factors but especially on tumor size and staging. Tumor grade is also important. High-grade tumors will have a poor prognosis, while low-grade tumors may go unnoticed for many years. The usual outcome is poor, because only 10% to 20% of hepatocellular carcinomas can be removed completely using surgery. If the cancer cannot be completely removed, the disease is usually deadly within 3 to 6 months.

Hepatocellular carcinoma, like any other cancer, develops when there is a mutation to the cellular machinery that causes the cell to replicate at a higher rate and/or results in the cell avoiding apoptosis. In particular, chronic viral infections of hepatitis B and/or C can aid the development of hepatocellular carcinoma by repeatedly causing the body's own immune system to attack the liver cells, some of which are infected by the virus, others merely bystanders. While this constant cycle of damage followed by repair can lead to mistakes during repair which in turn lead to carcinogenesis, this hypothesis is more applicable, at present, to hepatitis C. In hepatitis B, however, the integration of the viral genome into infected cells is the most consistently associated factor in malignancy. Alternatively, repeated consumption of large amounts of ethanol can have a similar effect.

Thus, within the scope of the present invention hepatitis B and/or C infections or hepatic cirrhosis are not merely to be considered as risk factors for tumor etiology but as early/intermediate stages in tumor progression (i.e. "pre-cancerous states") that are associated with hyper-proliferative tissue growth resulting in (often benign) non-invasive neoplasm which, in turn, may progress to malignant tumors such as HCC.

Such malignant tumors invade other tissues and often metastasize given enough time to do so. Malignant cells are often characterized by progressive and uncontrolled growth. Macroscopically, HCC appears as a nodular or infiltrative tumor. The nodular type may be solitary (having a large mass) or multiple (when developed as a complication of cirrhosis). Tumor nodules are round to oval, well circumscribed but not encapsulated. The diffuse type is poorly circumscribed and infiltrates the portal veins, rarely the hepatic veins.

Malignant tumors can spread (in form of metastasis) locally or through the blood stream and lymphatic system to other parts of the body. Liver is a common site for metastasis due to its dual blood supply (the liver receives blood via the hepatic artery and portal vein). Thus, tumor tissue (metastases) may form an embolus, that is, an object migrating from one part of the body (through circulation) and causes a blockage (occlusion) of a blood vessel in another part of the body. Such secondary hepatocellular tumors resulting from tumor embolism are referred herein as "carcinoma embolus".

The mammalian target cells employed in the present invention may be of human or non-human origin. However, the invention is typically performed with human cells. The term "one or more cells", as used herein, is to be understood not only to include individual cells but also tissues, organs, and organisms. The term "target cell", as used herein, refers to a cell being at least supposed to exhibit or to have a predisposition to develop hepatocellular cancer, whereas the term "control cell" typically denotes a (healthy) wild-type cell not having characteristics of such a cancerous phenotype. However, in some applications, for example, when comparing cells exhibiting different cancerous or pre-cancerous states, the cells having the less severe disease characteristics are typically considered the "control cells".

Typically, the target and control cells used are derived from biological samples collected from the subjects to be diagnosed for the presence or the predisposition to develop hepatocellular cancer. Furthermore, in order to corroborate the data obtained "comparative samples" may also be collected from subjects having a given known disease state. The biological samples may include body tissues and fluids, such as blood, sputum, and urine. Furthermore, the biological sample may contain a cell extract derived from or a cell population including a hepatic cell, preferably a cancerous hepatic cell or a hepatic cell derived from tissue suspected to be cancerous. Even more preferably the biological sample comprises a cell population derived from a hepatic tissue. Furthermore, the cell may be purified from the obtained body tissues and fluids if necessary, and then used as the biological sample. According to the present invention, the expression level of the nucleic acid markers of the present invention is determined in the subject-derived biological sample(s).

The sample used for detection in the *in vitro* methods of the present invention should generally be collected in a clinically acceptable manner, preferably in a way that nucleic acids (in particular RNA) or proteins are preserved. The samples to be analyzed are typically hepatic biopsies or resections. Furthermore, hepatocellular carcinoma cells may migrate into other tissues. Hence, blood and other types of sample can be used as well. A biopsy or resection may contain only a minority of (pre-)carcinoma cells. To increase the signal/background ratio, a resection can be divided into different sub-samples prior to analysis (for example, by laser-capture microdissection). Even if the total number of carcinoma cells in the biopsy or resection is limited, at least one of the sub-samples may contain an increased ratio of (pre-)cancerous *versus* non-cancerous cells. Samples, in particular after initial processing may be pooled. However, also non-pooled samples may be used.

The term "microRNA" (or "miRNA"), as used herein, is given its ordinary meaning in the art (reviewed, e.g. in Bartel, D.P. (2004) Cell 23, 281-292; He, L. and Hannon, G.J. (2004) Nat. Rev. Genet. 5, 522-531). Accordingly, a "microRNA" denotes a RNA molecule derived from a genomic locus that is processed from transcripts that can form local RNA precursor miRNA structures. The mature miRNA is usually 20, 21, 22, 23, 24, or 25 nucleotides in length, although other numbers of nucleotides may be present as well, for example 18, 19, 26 or 27 nucleotides.

The miRNA encoding sequence has the potential to pair with flanking genomic sequences, placing the mature miRNA within an imperfect RNA duplex (herein also referred to as stem-loop or hairpin structure or as pre-miRNA), which serves as an intermediate for miRNA processing from a longer precursor transcript. This processing typically occurs through the consecutive action of two specific endonucleases termed Drosha and Dicer, respectively. Drosha generates from the primary transcript (herein also denoted "pri-miRNA") a miRNA precursor (herein also denoted "pre-miRNA") that typically folds into a hairpin or stem-loop structure. From this miRNA precursor a miRNA duplex is excised by means of Dicer that comprises the mature miRNA at one arm of the hairpin or stem-loop structure and a similar-sized segment (commonly referred to miRNA*) at the other arm. The miRNA is then guided to its target mRNA to exert its function, whereas the miRNA* is degraded. In addition, miRNAs are typically derived from a segment of the genome that is distinct from predicted protein-coding regions.

The term "miRNA precursor" (or "precursor miRNA" or "pre-miRNA") as used herein, refers to the portion of a miRNA primary transcript from which the mature miRNA is processed. Typically, the pre-miRNA folds into a stable hairpin (i.e. a duplex) or a stem-loop structure. The hairpin structures typically range from 50 to 80 nucleotides in length, preferably from 60 to 70 nucleotides (counting the miRNA residues, those pairing to the miRNA, and any intervening segment(s) but excluding more distal sequences).

The term "nucleic acid molecule encoding a microRNA sequence", as used herein, denotes any nucleic acid molecule coding for a microRNA (miRNA). Thus, the term does not only refer to mature miRNAs but also to the respective precursor miRNAs and primary miRNA transcripts as defined above. Furthermore, the present invention is not restricted to RNA molecules but also includes corresponding DNA molecules encoding a microRNA, e.g. DNA molecules generated by reverse transcribing a miRNA sequence. A nucleic acid molecule encoding a microRNA sequence according to the invention typically encodes a single miRNA sequence (i.e. an individual miRNA). However, it is also possible that such nucleic acid molecule encodes two or more miRNA sequences (i.e. two or more miRNAs), for example a transcriptional unit comprising two or more miRNA sequences under the control of common regulatory sequences such as a promoter or a transcriptional terminator.

The term "nucleic acid molecule encoding a microRNA sequence", as used herein, is also to be understood to include "sense nucleic acid molecules" (i.e. molecules whose nucleic acid sequence (5'→ 3') matches or corresponds to the encoded miRNA (5'→ 3') sequence) and "anti-sense nucleic acid molecules" (i.e. molecules whose nucleic acid sequence is complementary to the encoded miRNA (5'→ 3') sequence or, in other words, matches the reverse complement (3'→ 5') of the encoded miRNA sequence). The term "complementary", as used herein, refers to the capability of an "anti-sense" nucleic acid molecule sequence of forming base pairs, preferably Watson-Crick base pairs, with the corresponding "sense" nucleic acid molecule sequence (having a sequence complementary to the anti-sense sequence).

Within the scope of the present invention, two nucleic acid molecules (i.e. the "sense" and the "anti-sense" molecule) may be perfectly complementary, that is, they do not contain any base mismatches and/or additional or missing nucleotides. Alternatively, the two molecules comprise one or more base mismatches or differ in their total numbers of nucleotides (due to additions or deletions). Preferably, the "complementary" nucleic acid molecule comprises at least ten contiguous nucleotides showing perfect complementarity with a sequence comprised in corresponding "sense" nucleic acid molecule.

Accordingly, the plurality of nucleic acid molecules encoding a miRNA sequence that are comprised in a diagnostic kit of the present invention may include one or more "sense nucleic acid molecules" and/or one or more "anti-sense nucleic acid molecules". In case, the diagnostic kit includes one or more "sense nucleic acid molecules" (i.e. the miRNA sequences as such), said molecules are to be considered to constitute the totality or at least a subset of differentially expressed miRNAs (i.e. molecular markers) being indicative for the presence of or the disposition to develop a particular condition, here hepatocellular cancer. On the other hand, in case a diagnostic kit includes one or more "anti-sense nucleic acid molecules" (i.e. sequences complementary to the miRNA sequences), said molecules may comprise *inter alia* probe molecules (for performing hybridization assays) and/or oligonucleotide primers (e.g., for reverse transcription or PCR applications) that are suitable for detecting and/or quantifying one or more particular (complementary) miRNA sequences in a given sample.

A plurality of nucleic acid molecules as defined within the present invention may comprise at least two, at least ten, at least 50, at least 100, at least 200, at least 500, at least 1.000, at least 10.000 or at least 100.000 nucleic acid molecules, each molecule encoding a miRNA sequence.

The term "differentially expressed", as used herein, denotes an altered expression level of a particular miRNA in the target cells as compared to the healthy control cells, which may be an up-regulation (i.e. an increased miRNA concentration in the target cells) or a down-regulation (i.e. a reduced or abolished miRNA concentration in the target cells). In other words, the nucleic acid molecule is activated to a higher or lower level in the target cells than in the control cells.

Within the scope of the present invention, a nucleic acid molecule is to considered differentially expressed if the respective expression levels of this nucleic acid molecule in target cells and control cells typically differ by at least 5% or at least 10%, preferably by at least 20% or at least 25%, and most preferably by at least 30% or at least 50%. Thus, the latter values correspond to an at least 1.3-fold or at least 1.5-fold up-regulation of the expression level of a given nucleic acid molecule in the target cells compared to the wild-type control cells or *vice versa* an at least 0.7-fold or at least 0.5-fold down-regulation of the expression level in the target cells, respectively.

The term "expression level", as used herein, refers to extent to which a particular miRNA sequence is transcribed from its genomic locus, that is, the concentration of a miRNA in the one or more cells to be analyzed.

As outlined above, the term "control cell" typically denotes a (healthy) wild-type cell not having characteristics of a HCC phenotype. However, in some applications, for example, when comparing cells exhibiting different cancerous or pre-cancerous states, the cells having the less severe disease characteristics are typically considered the "control cells".

The determining of expression levels typically follows established standard procedures well known in the art (cf., for example, Sambrook, J. et al. (1989) Molecular Cloning: A Laboratory Manual. 2nd Ed., Cold Spring Harbor Laboratory Press, Cold Spring Harbor, NY; Ausubel, F.M. et al. (2001) Current Protocols in Molecular Biology. Wiley & Sons, Hoboken, NJ). Determination may occur at the RNA level, for example by Northern blot analysis using miRNA-specific probes, or at the DNA level following reverse transcription (and cloning) of the RNA population, for example by quantitative PCR or real-time PCR techniques. The term "determining", as used herein, includes the analysis of any nucleic acid molecules encoding a microRNA sequence as described above. However, due to the short half-life of pri-miRNAs and pre-mRNAs typically the concentration of only the mature miRNA is measured.

In specific embodiments, the standard value of the expression levels obtained in several independent measurements of a given sample (for example, two, three, five or ten measurements) and/or several measurements within a population of target cells or control cells is used for analysis. The standard value may be obtained by any method known in the art. For example, a range of mean ± 2 SD (standard deviation) or mean ± 3 SD may be used as standard value.

The difference between the expression levels obtained for one or more target cells and one or more control cells may be normalized to the expression level of further control nucleic acids, e.g. housekeeping genes whose expression levels are known not to differ depending on the disease states of the cell. Exemplary housekeeping genes include *inter alia* β-actin, glycerinaldehyde 3-phosphate dehydrogenase, and ribosomal protein P1. In preferred embodiments, the control nucleic acid is another miRNA known to be stably expressed during the various non-cancerous and (pre-)cancerous states of the cell.

However, instead of determining in any experiment the expression levels for one or more control cells it may also be possible to define based on experimental evidence and/or prior art data on or more cut-off values for a particular cell phenotype (i.e. a disease state). In such scenario, the respective expression levels for the one or more target cells can be determined by using a stably expressed control miRNA for normalization. If the "normalized" expression levels calculated are higher than the respective cutoff value defined, then this finding would be indicative for an up-regulation of gene expression. *Vice versa*, if the "normalized" expression levels calculated are lower than the respective cutoff value defined, then this finding would be indicative for a down-regulation of gene expression.

In the context of the present invention, the term "identifying one or more mammalian target cells exhibiting or having a predisposition to develop hepatocellular cancer" is intended to also encompass predictions and likelihood analysis (in the sense of "diagnosing"). The compositions and methods disclosed herein are intended to be used clinically in making decisions concerning treatment modalities, including therapeutic intervention, diagnostic criteria such as disease stages, and disease monitoring and surveillance for the disease. According to the present invention, an intermediate result for examining the condition of a subject may be provided. Such intermediate result may be combined with additional information to assist a doctor, nurse, or other practitioner to diagnose that a subject suffers from the disease. Alternatively, the invention may be used to detect cancerous cells in a subject-derived tissue, and provide a doctor with useful information for diagnosis. Furthermore, the invention may also be used to discriminate between different types of hepatocellular tumors.

Within the present invention, one or more differentially expressed nucleic acid molecules identified together represent a nucleic acid expression signature that is indicative for the presence of or the predisposition to develop hepatocellular cancer in the target cells. The term "expression signature", as used herein, denotes a set of nucleic acid molecules (e.g., miRNAs), wherein the expression level of the individual nucleic acid molecules differs between the (cancerous) target cells and the (non-cancerous) control cells. Herein, a nucleic acid expression signature is also referred to as a set of markers and represents a minimum number of (different) nucleic acid molecules, each encoding a miRNA sequence that is capable for identifying a phenotypic state of a target cell.

In specific embodiments, the nucleic acid expression signature comprises at least five nucleic acid molecules, each encoding a (different) miRNA sequence. Preferably, the nucleic acid expression signature comprises at least eight or at least ten (different) nucleic acid molecules. Particularly preferably, the nucleic acid signature comprises at least twelve or at least fifteen (different) nucleic acid molecules.

Typically, the nucleic acid molecules comprised in the nucleic acid expression signature are human sequences (hereinafter designated "hsa" (Homo sapiens)).

In further preferred embodiments, the nucleic acid expression signature comprises at least one nucleic acid molecule encoding a miRNA sequence whose expression is up-regulated (i.e. its concentration is increased) in the one or more target cells compared to the one or more control cells and at least one nucleic acid molecule encoding a miRNA sequence whose expression is down-regulated (i.e. its concentration is reduced) in the one or more target cells compared to the one or more control cells.

In preferred embodiments of the invention, the nucleic acid expression signature of the diagnostic kit comprises any one or more human target cell-derived nucleic acid molecules encoding microRNA sequences selected from the group consisting of hsa-mir-221 (SEQ ID NO:1), hsa-mir-324-5p (SEQ ID NO:2), hsa-mir-96 (SEQ ID NO: 3), hsa-mir-18a (SEQ ID NO:4), hsa-mir-18b (SEQ ID NO:5), hsa-mir-30d (SEQ ID NO:6), hsa-mir-331-3p (SEQ ID NO:7), hsa-mir-183 (SEQ ID NO:8), hsa-mir-551b (SEQ ID NO:9), hsa-mir-139-Sp (SEQ ID NO:10), hsa-mir-144 (SEQ ID NO:11), hsa-mir-144* (SEQ ID NO:12), and hsa-mir-450a (SEQ ID NO:13).

The nucleic acid sequences of the above-referenced miRNAs are listed in Table 1.

**TABLE 1**

| **miRNA** | **Sequence (5'→ 3')** |
|---|---|
| hsa-miR-221 | agcuacauugucugcuggguuuc |
| hsa-miR-324-5p | cgcauccccuagggcauuggugu |
| hsa-miR-96 | uuuggcacuagcacauuuuugcu |
| hsa-miR-18a | uaaggugcaucuagugcagauag |
| hsa-miR-18b | uaaggugcaucuagugcaguuag |
| hsa-miR-30d | uguaaacauccccgacuggaag |
| hsa-miR-331-3p | gccccugggccuauccuagaa |
| hsa-miR-183 | uauggcacugguagaauucacu |
| hsa-miR-551b | gcgacccauacuugguuucag |
| hsa-miR-139-5p | ucuacagugcacgugucuccag |
| hsa-miR-144 | uacaguauagaugauguacu |
| hsa-miR-144* | ggauaucaucauauacuguaag |
| hsa-miR-450a | uuuugcgauguguuccuaauau |

All miRNA sequences disclosed herein have been deposited in the miRBase database (http://microrna.sanger.ac.uk/; see also Griffiths-Jones S. et al. (2008) Nucl. Acids Res. 36, D154-D158).

Particularly preferably, the expression of any one or more of the nucleic acid molecules encoding hsa-mir-221, hsa-mir-324-5p, hsa-mir-96, hsa-mir-18a, hsa-mir-18b, hsa-mir-30d, hsa-mir-331-3p, hsa-mir-183, and hsa-mir-551b is up-regulated and the expression of any one or more of the nucleic acid molecules hsa-mir-139-5p, hsa-mir-144, hsa-mir-144*, and hsa-mir-450a is down-regulated in the in the one or more target cells compared to the one or more control cells.

The terms "one or more of the plurality of nucleic acid molecules" and "any one or more human target cell-derived nucleic acid molecules", as used herein, may relate to any subgroup of the plurality of nucleic acid molecules, e.g., any one, any two, any three, any four, any five, any six, any seven, any eight, any nine, any ten, and so forth nucleic acid molecules, each encoding a microRNA sequence that are comprised in the nucleic acid expression signature, as defined herein.

In further embodiments of the invention, the nucleic acid expression signature includes at least any one or more nucleic acid molecules encoding the miRNAs specified above and also contains one or more additional nucleic acid molecules encoding any further miRNA sequences that are differentially expressed in the target cells and in one or more control cells analyzed, particularly any one or more human target cell-derived nucleic acid molecules encoding microRNA sequences selected from the group consisting ofhsa-mir-100 (SEQ ID NO:14), hsa-mir-101 (SEQ ID NO:15), hsa-mir-10a (SEQ ID NO: 16), hsa-mir-139-3p (SEQ ID NO:17), hsa-mir-148a (SEQ ID NO:18), hsa-mir-30c (SEQ ID NO:19), hsa-mir-122* (SEQ ID NO:20), hsa-mir-152 (SEQ ID NO:21), hsa-mir-24-1* (SEQ ID NO:22), hsa-mir-26b (SEQ ID NO:23), hsa-mir-29c* (SEQ ID NO:24), hsa-mir-30e (SEQ ID NO:25), hsa-mir-30e* (SEQ ID NO:26), hsa-mir-378 (SEQ ID NO: 27), hsa-mir-378* (SEQ ID NO:28), hsa-mir-424 (SEQ ID NO:29), hsa-mir-455-3p (SEQ ID NO:30), hsa-mir-455-5p (SEQ ID NO:31), hsa-mir-505 (SEQ ID NO:32), hsa-mir-542-3p (SEQ ID NO:33), hsa-mir-99a (SEQ ID NO:34), hsa-mir-29c (SEQ ID NO:35), and hsa-mir-338-3p (SEQ ID NO:36).

Preferably, the expression of any of these nucleic acid molecules (SEQ ID NO:14 to SEQ ID NO:36) is down-regulated in the in the one or more target cells compared to the one or more control cells.

The above-mentioned miRNAs (SEQ ID NO: 1 to SEQ ID NO:36) were found to differentially expressed in several types of hepatocellular cancer tested as compared to control cells, particularly in hepatocellular carcinoma (i.e. a primary liver malignancy) and in carcinoma embolus (i.e. a secondary malignancy). Thus the above expression signatures appear to be indicative for "hepatocellular tumor invasiveness" independent of the type of tumor.

In further specific embodiments of the invention, the diagnostic kit is for the further use of discriminating different types of hepatocellular cancer, particularly for discriminating hepatocellular cancer selected from the group consisting of hepatocellular carcinoma and carcinoma embolus.

In preferred embodiments, the hepatocellular cancer is hepatocellular carcinoma, and the nucleic acid expression signature comprises any one or more of the nucleic acid molecules encoding hsa-miR-224 (SEQ ID NO:37), hsa-miR-532-3p (SE ID NO:38), hsa-miR-663 (SEQ ID NO:39), hsa-miR-99b (SEQ ID NO:40), hsa-miR-362-5p (SEQ ID NO: 41), hsa-miR-652 (SEQ ID NO:42), hsa-miR-222 (SEQ ID NO:43), hsa-miR-501-3p (SEQ ID NO:44), hsa-miR-375 (SEQ ID NO:45), hsa-miR-451 (SEQ ID NO:46), and hsa-miR-335 (SEQ ID NO: 47).

Within the scope of the present invention, the expression signature for discriminating hepatocellular carcinoma may comprise any one or more of the nucleic acid molecules identified as SEQ ID NO:37 to SEQ ID NO:47 but none of the before-mentioned nucleic acid molecules identified as SEQ ID NO:1 to SEQ ID NO:36. However, it may also be possible that the expression signature for discriminating hepatocellular carcinoma may comprise any one or more of the nucleic acid molecules identified as SEQ ID NO:37 to SEQ ID NO:47 and in addition any one or more of the before-mentioned nucleic acid molecules identified as SEQ ID NO:1 to SEQ ID NO:36.

The nucleic acid sequences of the above-referenced miRNAs are listed in Table 2.

**TABLE 2**

| **miRNA** | **Sequence (5' → 3')** |
|---|---|
| hsa-miR-224 | caagucacuagugguuccguu |
| hsa-miR-532-3p | ccucccacacccaaggcuugca |
| hsa-miR-663 | aggcggggcgccgcgggaccgc |
| hsa-miR-99b | cacccguagaaccgaccuugcg |
| hsa-miR-362-5p | aauccuuggaaccuaggugugagu |
| hsa-miR-652 | aauggcgccacuaggguugug |
| hsa-miR-222 | agcuacaucuggcuacugggu |
| hsa-miR-501-3p | aaugcacccgggcaaggauucu |
| hsa-miR-375 | uuuguucguucggcucgcguga |
| hsa-miR-451 | aaaccguuaccauuacugaguu |
| hsa-miR-335 | ucaagagcaauaacgaaaaaugu |

Particularly preferably, the expression of any one or more of the nucleic acid molecules encoding hsa-miR-224, hsa-miR-532-3p, hsa-miR-663, hsa-miR-99b, hsa-miR-362-5p, hsa-miR-652, hsa-miR-222, and hsa-miR-501-3p is up-regulated and the expression of any one or more of the nucleic acid molecules hsa-miR-375, hsa-miR-451, and hsa-miR-335 is down-regulated in the in the one or more target cells compared to the one or more control cells.

In further embodiments of the invention, the nucleic acid expression signature for discriminating hepatocellular carcinoma includes at least any one or more nucleic acid molecules encoding the miRNAs specified above (SEQ ID NO:37 to SEQ ID NO:47) and also contains one or more additional nucleic acid molecules encoding any further miRNA sequences that are differentially expressed in the target cells and in one or more control cells analyzed, particularly any one or more human target cell-derived nucleic acid molecules encoding microRNA sequences selected from the group consisting ofhsa-miR-130b (SEQ ID NO:48), hsa-miR-132 (SEQ ID NO:49), hsa-miR-106b (SEQ ID NO:50), hsa-miR-125a-5p (SEQ ID NO:51), hsa-miR-151-3p (SEQ ID NO:52), hsa-miR-17 (SEQ ID NO:53), hsa-miR-21 * (SEQ ID NO: 54), hsa-miR-25 (SEQ ID NO:55), hsa-miR-301a (SEQ ID NO:56), hsa-miR-423-5p (SEQ ID NO:57), hsa-miR-425 (SEQ ID NO:58), hsa-miR-484 (SEQ ID NO:59), hsa-miR-500* (SEQ ID NO:60), hsa-miR-502-3p (SEQ ID NO:61), hsa-miR-502-5p (SEQ ID NO:62), hsa-miR-532-5p (SEQ ID NO:63), hsa-miR-769-5p (SEQ ID NO:64), hsa-miR-93 (SEQ ID NO:65), hsa-miR-181b (SEQ ID NO:66), hsa-miR-501-5p (SEQ ID NO:67), hsa-miR-194 (SEQ ID NO:68), hsa-miR-199b-3p (SEQ ID NO:69), and hsa-miR-215 (SEQ ID NO:70).

Preferably, the expression of any one or more of the nucleic acid molecules encoding hsa-miR-130b, hsa-miR-132, hsa-miR-106b, hsa-miR-125a-5p, hsa-miR-151-3p, hsa-miR-17, hsa-miR-21*, hsa-miR-25, hsa-miR-301a, hsa-miR-423-5p, hsa-miR-425, hsa-miR-484, hsa-miR-500* , hsa-miR-502-3p, hsa-miR-502-5p, hsa-miR-532-5p, hsa-miR-769-5p, hsa-miR-93, hsa-miR-181b, and hsa-miR-501-5p is up-regulated and the expression of any one or more of the nucleic acid molecules hsa-miR-194, hsa-miR-199b-3p, hsa-miR-215 is down-regulated in the in the one or more target cells compared to the one or more control cells.

In other preferred embodiments, the hepatocellular cancer is carcinoma embolus, and the nucleic acid expression signature comprises any one or more of the nucleic acid molecules encoding hsa-miR-199b-5p (SEQ ID NO:71), hsa-miR-2 (SEQ ID NO:72), hsa-miR-19b (SEQ ID NO:73), hsa-miR-103 (SEQ ID NO:74), hsa-miR-23a (SEQ ID NO:75), hsa-miR-125b-2* (SEQ ID NO:76), hsa-miR-30c-2* (SEQ ID NO:77), hsa-miR-505* (SEQ ID NO:78), and hsa-miR-203 (SEQ ID NO:79).

The nucleic acid sequences of the above-referenced miRNAs are listed in Table 3.

**TABLE 3**

| **miRNA** | **Sequence (5' → 3')** |
|---|---|
| hsa-miR-199b-5p | cccaguguuuagacuaucuguuc |
| hsa-miR-21 | uagcuuaucagacugauguuga |
| hsa-miR-19b | ugugcaaauccaugcaaaacuga |
| hsa-miR-103 | agcagcauuguacagggcuauga |
| hsa-miR-23a | aucacauugccagggauuucc |
| hsa-miR-125b-2* | ucacaagucaggcucuugggac |
| hsa-miR-30c-2* | cugggagaaggcuguuuacucu |
| hsa-miR-505* | gggagccaggaaguauugaugu |
| hsa-miR-203 | gugaaauguuuaggaccacuag |

Within the scope of the present invention, the expression signature for discriminating carcinoma embolus may comprise any one or more of the nucleic acid molecules identified as SEQ ID NO:71 to SEQ ID NO:79 but none of the before-mentioned nucleic acid molecules identified as SEQ ID NO:1 to SEQ ID NO:36. However, it may also be possible that the expression signature for discriminating carcinoma embolus may comprise any one or more of the nucleic acid molecules identified as SEQ ID NO:71 to SEQ ID NO:79 and in addition any one or more of the before-mentioned nucleic acid molecules identified as SEQ ID NO:1 to SEQ ID NO:36.

Particularly preferably, the expression of any one or more of the nucleic acid molecules encoding hsa-miR-199b-5p, hsa-miR-21, hsa-miR-19b, hsa-miR-103, and hsa-miR-23a is up-regulated and the expression of any one or more of the nucleic acid molecules hsa-miR-125b-2*, hsa-miR-30c-2*, hsa-miR-505*, and hsa-miR-203 is down-regulated in the in the one or more target cells compared to the one or more control cells.

In further embodiments of the invention, the nucleic acid expression signature for discriminating carcinoma embolus includes at least any one or more nucleic acid molecules encoding the miRNAs specified above (SEQ ID NO:71 to SEQ ID NO:79) and also contains one or more additional nucleic acid molecules encoding any further miRNA sequences that are differentially expressed in the target cells and in one or more control cells analyzed, particularly any one or more human target cell-derived nucleic acid molecules encoding microRNA sequences selected from the group consisting ofhsa-let-7c (SEQ ID NO:80), hsa-miR-122 (SEQ ID NO:81), hsa-miR-125b (SEQ ID NO:82), hsa-miR-130a (SEQ ID NO:83), hsa-miR-195 (SEQ ID NO:84), hsa-miR-30a (SEQ ID NO:85), hsa-miR-126* (SEQ ID NO:86), hsa-miR-128 (SEQ ID NO:87), hsa-miR-192* (SEQ ID NO:88), hsa-miR-28-5p (SEQ ID NO:89), hsa-miR-30a* (SEQ ID NO:90), hsa-miR-30b* (SEQ ID NO:91), hsa-miR-486-5p (SEQ ID NO:92), hsa-miR-497 (SEQ ID NO:93), hsa-miR-542-5p (SEQ ID NO:94), hsa-miR-564 (SEQ ID NO:95), hsa-miR-590-5p (SEQ ID NO:96), hsa-miR-340 (SEQ ID NO:97), hsa-miR-582-5p (SEQ ID NO:98), and hsa-miR-194* (SEQ ID NO:99).

Preferably, the expression of any of these nucleic acid molecules (SEQ ID NO:80 to SEQ ID NO:99) is down-regulated in the in the one or more target cells compared to the one or more control cells.

In a second aspect, the present invention relates to a method for identifying one or more mammalian target cells exhibiting or having a predisposition to develop hepatocellular cancer, the method comprising:
(a) determining in the one or more target cells the expression levels of a plurality of nucleic acid molecules, each nucleic acid molecule encoding a microRNA sequence;
(b) determining the expression levels of the plurality of nucleic acid molecules in one or more control cells; and
(c) identifying from the plurality of nucleic acid molecules one or more nucleic acid molecules that are differentially expressed in the target and control cells by comparing the respective expression levels obtained in steps (a) and (b),
wherein the one or more differentially expressed nucleic acid molecules together represent a nucleic acid expression signature, as defined herein, that is indicative for the presence of or the predisposition to develop hepatocellular cancer.

In preferred embodiments of the invention, the method is for the further use of discriminating hepatocellular cancer selected from the group consisting of hepatocellular carcinoma and carcinoma embolus.

The method of the present invention comprises determining and comparing the expression levels of a plurality of nucleic acid molecules encoding a microRNA sequence both in one or more target cells supposed to exhibit or to have a predisposition to develop hepatocellular cancer and in one or more control cells, i.e. typically wild-type cells not showing the characteristics of such a cancerous phenotype (cf. also the discussion above).

In a third aspect, the invention relates to a method for preventing or treating hepatocellular cancer in one or more mammalian target cells, the method comprising:
(a) identifying in one or more target cells a nucleic acid expression signature by using a method, as defined herein; and
(b) modifying in the one or more cells the expression of one or more nucleic acid molecules encoding a microRNA sequence that is/are comprised in the nucleic acid expression signature in such way that the expression of a nucleic acid molecule whose expression is up-regulated in the one or more target cells is down-regulated and the expression of a nucleic acid molecule whose expression is down-regulated in the one or more target cells is up-regulated.

The term "modifying the expression of a nucleic acid molecule encoding a miRNA sequence", as used herein, denotes any manipulation of a particular nucleic acid molecule resulting in an altered expression level of said molecule, that is, the production of a different amount of corresponding miRNA as compared to the expression of the "wild-type" (i.e. the unmodified control). The term "different amount", as used herein, includes both a higher amount and a lower amount than determined in the unmodified control. In other words, a manipulation, as defined herein, may either up-regulate (i.e. activate) or down-regulate (i.e. inhibit) the expression (i.e. particularly transcription) of a nucleic acid molecule.

Within the present invention, expression of one or more nucleic acid molecules encoding a microRNA sequence comprised in the nucleic acid expression signature is modified in such way that the expression of a nucleic acid molecule whose expression is up-regulated in the one or more target cells is down-regulated and the expression of a nucleic acid molecule whose expression is down-regulated in the one or more target cells is up-regulated. In other words, the modification of expression of a particular nucleic acid molecule encoding a miRNA sequence occurs in an anti-cyclical pattern to the regulation of said molecule in the one or more cancerous target cells in order to interfere with the "excess activity" of an up-regulated molecule and/or to restore the "deficient activity" of a down-regulated molecule in the one or more target cells.

In a preferred embodiment of the inventive method, down-regulating the expression of a nucleic acid molecule comprises introducing into the one or more target cells a nucleic acid molecule encoding a sequence that is complementary to the microRNA sequence encoded by nucleic acid molecule to be down-regulated.

The term "introducing into a cell", as used herein, refers to any manipulation allowing the transfer of one or more nucleic acid molecules into a cell. Examples of such techniques include *inter alia* transfection or transduction techniques all of them well established in the art (cf., for example, Sambrook, J. et al. (1989) Molecular, Cloning: A Laboratory Manual, 2nd ed., Cold Spring Harbor Laboratory Press, Cold Spring Harbor, NY; Ausubel, F.M. et al. (2001) Current Protocols in Molecular Biology, Wiley & Sons, Hoboken, NJ).

The term "complementary sequence", as used herein, is to be understood that the "complementary" nucleic acid molecule (herein also referred to as an "anti-sense nucleic acid molecule") introduced into the one or more cells is capable of forming base pairs, preferably Watson-Crick base pairs, with the up-regulated endogenous "sense" nucleic acid molecule.

Two nucleic acid molecules (i.e. the "sense" and the "anti-sense" molecule) may be perfectly complementary, that is, they do not contain any base mismatches and/or additional or missing nucleotides. In other embodiments, the two molecules comprise one or more base mismatches or differ in their total numbers of nucleotides (due to additions or deletions). In further embodiments, the "complementary" nucleic acid molecule comprises a stretch of at least ten contiguous nucleotides showing perfect complementarity with a sequence comprised in the up-regulated "sense" nucleic acid molecule.

The "complementary" nucleic acid molecule (i.e. the nucleic acid molecule encoding a nucleic acid sequence that is complementary to the microRNA sequence encoded by nucleic acid molecule to be down-regulated) may be a naturally occurring DNA- or RNA molecule or a synthetic nucleic acid molecule comprising in its sequence one or more modified nucleotides which may be of the same type or of one or more different types.

For example, it may be possible that such a nucleic acid molecule comprises at least one ribonucleotide backbone unit and at least one deoxyribonucleotide backbone unit. Furthermore, the nucleic acid molecule may contain one or more modifications of the RNA backbone into 2'-O-methyl group or 2'-O-methoxyethyl group (also referred to as "2'-O-methylation"), which prevented nuclease degradation in the culture media and, importantly, also prevented endonucleolytic cleavage by the RNA-induced silencing complex nuclease, leading to irreversible inhibition of the miRNA. Another possible modification - which is functionally equivalent to 2'-O-methylation - involves locked nucleic acids (LNAs) representing nucleic acid analogs containing one or more LNA nucleotide monomers with a bicyclic furanose unit locked in an RNA-mimicking sugar conformation (cf., e.g., Orom, U.A. et al. (2006) Gene 372, 137-141).

Another class of silencers of miRNA expression was recently developed. These chemically engineered oligonucleotides, named "antagomirs", represent single-stranded 23-nucleotide RNA molecules conjugated to cholesterol (Krutzfeldt, J. et al. (2005) Nature 438, 685-689). As an alternative to such chemically modified oligonucleotides, microRNA inhibitors that can be expressed in cells, as RNAs produced from transgenes, were generated as well. Termed "microRNA sponges", these competitive inhibitors are transcripts expressed from strong promoters, containing multiple, tandem binding sites to a microRNA of interest (Ebert, M.S. et al. (2007) Nat. Methods 4, 721-726).

In particularly preferred embodiments of the inventive method, the one or more nucleic acid molecules whose expression is to be down-regulated encode microRNA sequences selected from:
(a) the group consisting of hsa-miR-221, hsa-miR-324-5p, hsa-miR-96, hsa-miR-18a, hsa-miR-18b, hsa-miR-30d, hsa-miR-331-3p, hsa-miR-183, and hsa-miR-551b with respect to the "general" expression signature, presumably indicative for tumor invasiveness as defined above; and/or
(b) the group consisting of hsa-miR-224, hsa-miR-532-3p, hsa-miR-663, hsa-miR-99b, hsa-miR-362-5p, hsa-miR-652, hsa-miR-222, and hsa-miR-501-3p with respect to the expression signature for discriminating hepatocellular carcinoma as defined above; and/or.
(c) the group consisting of hsa-miR-199b-5p, hsa-miR-21, hsa-miR-19b, hsa-miR-103, and hsa-miR-23a with respect to the expression signature for discriminating carcinoma embolus as defined above.

In a further preferred embodiment of the inventive method, up-regulating the expression of a nucleic acid molecule comprises introducing into the one or more target cells a nucleic acid molecule encoding the microRNA sequence encoded by nucleic acid molecule to be up-regulated. In other words, the up-regulation of the expression of a nucleic acid molecule encoding a miRNA sequence is accomplished by introducing into the one or more cells another copy of said miRNA sequence (i.e. an additional "sense" nucleic acid molecule). The "sense" nucleic acid molecule to be introduced into the one or more target cells may comprise the same modification as the "anti-sense" nucleic acid molecules described above.

In a particularly preferred embodiment, the one or more nucleic acid molecules whose expression is to be up-regulated encode microRNA sequences selected from:
(a) the group consisting of hsa-miR-139-5p, hsa-miR-144, hsa-miR-144*, and hsa-miR-450a with respect to the "general" expression signature, presumably indicative for tumor invasiveness as defined above; and/or
(b) the group consisting of hsa-miR-375, hsa-miR-451, and hsa-miR-335 with respect to the expression signature for discriminating hepatocellular carcinoma as defined above; and/or.
(c) the group consisting of hsa-miR-125b-2*, hsa-miR-30c-2*, hsa-miR-505*, and hsa-miR-203 with respect to the expression signature for discriminating carcinoma embolus as defined above.

The "sense" and/or the "anti-sense" nucleic acid molecules to be introduced into the one ore more target cells in order to modify the expression of one or more nucleic acid molecules encoding a microRNA sequence that is/are comprised in the nucleic acid expression signature may be operably linked to a regulatory sequence in order to allow expression of the nucleotide sequence.

In order to unravel any potential implication of the miRNAs identified in the cancerous or pre-cancerous samples preliminary functional analyses may be performed with respect to the identification of mRNA target sequences to which the miRNAs may bind. Based on the finding that miRNAs may be involved in both tumor suppression and tumorigenesis (reviewed, e.g., in Esquela-Kerscher, A. and Slack, F.J (2006) *supra*; Calin, G.A. and Croce, C.M. (2007) *supra*; Blenkiron, C. and Miska, E.A. (2007) *supra*) it is likely to speculate that mRNA target sites for such miRNAs include tumor suppressor genes as well as oncogenes.

A nucleic acid molecule is referred to as "capable of expressing a nucleic acid molecule" or capable "to allow expression of a nucleotide sequence" if it comprises sequence elements which contain information regarding to transcriptional and/or translational regulation, and such sequences are "operably linked" to the nucleotide sequence encoding the polypeptide. An operable linkage is a linkage in which the regulatory sequence elements and the sequence to be expressed (and/or the sequences to be expressed among each other) are connected in a way that enables gene expression.

The precise nature of the regulatory regions necessary for gene expression may vary among species, but in general these regions comprise a promoter which, in prokaryotes, contains both the promoter *per se*, i.e. DNA elements directing the initiation of transcription, as well as DNA elements which, when transcribed into RNA, will signal the initiation of translation. Such promoter regions normally include 5' non-coding sequences involved in initiation of transcription and translation, such as the -35/-10 boxes and the Shine-Dalgarno element in prokaryotes or the TATA box, CAAT sequences, and 5'-capping elements in eukaryotes. These regions can also include enhancer or repressor elements as well as translated signal and leader sequences for targeting the native polypeptide to a specific compartment of a host cell. In addition, the 3' non-coding sequences may contain regulatory elements involved in transcriptional termination, polyadenylation or the like. If, however, these termination sequences are not satisfactory functional in a particular host cell, then they may be substituted with signals functional in that cell.

Furthermore, the expression of the nucleic molecules, as defined herein, may also be influenced by the presence, e.g., of modified nucleotides (cf. the discussion above). For example, locked nucleic acid (LNA) monomers are thought to increase the functional half-life of miRNAs *in vivo* by enhancing the resistance to degradation and by stabilizing the miRNA-target duplex structure that is crucial for silencing activity (cf., e.g., Naguibneva, I. et al. (2006) Biomed. Pharmacother. 60, 633-638).

Therefore, a nucleic acid molecule of the invention to be introduced into the one or more cells provided may include a regulatory sequence, preferably a promoter sequence, and optionally also a transcriptional termination sequence. The promoters may allow for either a constitutive or an inducible gene expression. Suitable promoters include *inter alia* the *E. coli lac*UV5 and *tet* (tetracycline-responsive) promoters, the T7 promoter as well as the SV40 promoter or the CMV promoter.

The nucleic acid molecules of the invention may also be comprised in a vector or other cloning vehicles, such as plasmids, phagemids, phages, cosmids or artificial chromosomes. In a preferred embodiment, the nucleic acid molecule is comprised in a vector, particularly in an expression vector. Such an expression vector can include, aside from the regulatory sequences described above and a nucleic acid sequence encoding a genetic construct as defined in the invention, replication and control sequences derived from a species compatible with the host that is used for expression as well as selection markers conferring a selectable phenotype on transfected cells. Large numbers of suitable vectors such as pSUPER and pSUPERIOR are known in the art, and are commercially available.

In a forth aspect, the invention relates to a pharmaceutical composition for the prevention and/or treatment of hepatocellular cancer, preferably manifested as an adenocarcinoma, in one or more mammalian target cells, the composition comprising one or more nucleic acid molecules, each nucleic acid molecule encoding a sequence that is at least partially complementary to a microRNA sequence encoded by a nucleic acid molecule whose expression is up-regulated in the one or more target cells, as defined herein, and/or that corresponds to a microRNA sequence encoded by a nucleic acid molecule whose expression is down-regulated in the one or more target cells, as defined herein.

In a final aspect, the invention is directed to the use of such a pharmaceutical composition for the manufacture of a medicament for the prevention and/or treatment of hepatocellular cancer, preferably manifested as an adenocarcinoma.

Within the scope of the present invention, suitable pharmaceutical compositions include *inter alia* those compositions suitable for oral, rectal, nasal, topical (including buccal and sub-lingual), peritoneal and parenteral (including intramuscular, subcutaneous and intravenous) administration, or for administration by inhalation or insufflation. Administration may be local or systemic. Preferably, administration is accomplished via the oral or intravenous routes. The formulations may also be packaged in discrete dosage units.

Pharmaceutical compositions according to the present invention include any pharmaceutical dosage forms established in the art, such as *inter alia* capsules, microcapsules, cachets, pills, tablets, powders, pellets, multi-particulate formulations (e.g., beads, granules or crystals), aerosols, sprays, foams, solutions, dispersions, tinctures, syrups, elixirs, suspensions, water-in-oil emulsions such as ointments, and oil-in water emulsions such as creams, lotions, and balms.

The ("sense" and "anti-sense") nucleic acid molecules described above can be formulated into pharmaceutical compositions using pharmacologically acceptable ingredients as well as established methods of preparation (Gennaro, A.L. and Gennaro, A.R. (2000) Remington: The Science and Practice of Pharmacy, 20th Ed., Lippincott Williams & Wilkins, Philadelphia, PA; Crowder, T.M. et al. (2003) A Guide to Pharmaceutical Particulate Science. Interpharm/CRC, Boca Raton, FL; Niazi, S.K. (2004) Handbook of Pharmaceutical Manufacturing Formulations, CRC Press, Boca Raton, FL).

In order to prepare the pharmaceutical compositions, pharmaceutically inert inorganic or organic excipients (i.e. carriers) can be used. To prepare e.g. pills, tablets, capsules or granules, for example, lactose, talc, stearic acid and its salts, fats, waxes, solid or liquid polyols, natural and hardened oils may be used. Suitable excipients for the production of solutions, suspensions, emulsions, aerosol mixtures or powders for reconstitution into solutions or aerosol mixtures prior to use include water, alcohols, glycerol, polyols, and suitable mixtures thereof as well as vegetable oils.

The pharmaceutical composition may also contain additives, such as, for example, fillers, binders, wetting agents, glidants, stabilizers, preservatives, emulsifiers, and furthermore solvents or solubilizers or agents for achieving a depot effect. The latter is to be understood that the nucleic acid molecules may be incorporated into slow or sustained release or targeted delivery systems, such as liposomes, nanoparticles, and microcapsules.

To target most tissues within the body, clinically feasible noninvasive strategies are required for directing such pharmaceutical compositions, as defined herein, into cells. In the past years, several approaches have achieved impressive therapeutic benefit following intravenous injection into mice and primates using reasonable doses of siRNAs without apparent limiting toxicities.

One approach involves covalently coupling the passenger strand (miRNA* strand) of the miRNA to cholesterol or derivatives/conjugates thereof to facilitate uptake through ubiquitously expressed cell-surface LDL receptors (Soutschek, J. et al. (2004) Nature 432, 173-178). Alternatively, unconjugated, PBS-formulated locked-nucleic-acid-modified oligonucleotides (LNA-antimiR) may be used for systemic delivery (Elmen, J. et al. (2008) Nature 452, 896-899). Another strategy for delivering miRNAs involves encapsulating the miRNAs into specialized liposomes formed using polyethylene glycol to reduce uptake by scavenger cells and enhance time spent in the circulation. These specialized nucleic acid particles (stable nucleic acid-lipid particles or SNALPs) delivered miRNAs effectively to the liver (and not to other organs (cf., e.g., Zimmermann, T.S. et al. (2006) Nature 441, 111-114). Recently, a new class of lipid-like delivery molecules, termed lipidoids (synthesis scheme based upon the conjugate addition of alkylacrylates or alkyl-acrylamides to primary or secondary amines) has been described as delivery agents for RNAi therapeutics (Akinc, A. et al. (2008) Nat. Biotechnol. 26, 561-569).

A further cell-specific targeting strategy involves the mixing of miRNAs with a fusion protein composed of a targeting antibody fragment linked to protamine, the basic protein that nucleates DNA in sperm and binds miRNAs by charge (Song, E. et al. (2005) Nat. Biotechnol. 23, 709-717). Multiple modifications or variations of the above basic delivery approaches have recently been developed. These techniques are known in the art and reviewed, e.g., in de Fougerolles, A. et al. (2007) Nat. Rev. Drug Discov. 6, 443-453; Kim, D.H. and Rossi, J.J. (2007) Nat. Genet. 8, 173-184).

The invention is further described by the figures and the following examples, which are solely for the purpose of illustrating specific embodiments of this invention, and are not to be construed as limiting the scope of the invention in any way.

### EXAMPLES

### Example 1: Sample collection and preparation

The principal method steps for identifying one or more target cells in a patient's sample exhibiting or having a predisposition to develop colorectal cancer are shown in Fig. 2.

Biopsies were taken during surgery (e.g., liver resection), if abnormal areas were found. Biopsy samples are usually about 0.5 cm in diameter. Surgical specimens were snap-frozen in liquid nitrogen at or immediately after collection. Samples may also be stored at -80°C.

Patient data (age, sex, imaging data, therapy, other medical conditions, family history, and the like) were derived from the hospital databases for matching the various samples collected. Pathologic follow-up (for example, histological analysis via hematoxylin and eosin (H&E) staining) was used for evidently determining the disease state (i.e. control, pre-cancerous stage (e.g., hepatic cirrhosis), primary malignancy (e.g., hepatocellular carcinoma) or secondary/metastatic malignancy (e.g., carcinoma embolus)) of a given sample as well as to ensure a consistent classification of the specimens.

Laser-capture micro-dissection was optionally performed for each cancerous sample in order to specifically isolate tumor cell populations (about 200.000 cells). In brief, a transparent transfer film is applied to the surface of a tissue section or specimen. Under a microscope, the thin tissue section is viewed through the glass slide on which it is mounted and clusters of cells are identified for isolation. When the cells of choice are in the center of the field of view, a near IR laser diode integral with the microscope optics is activated. The pulsed laser beam activates a spot on the transfer film, fusing the film with the underlying cells of choice. The transfer film with the bonded cells is then lifted off the thin tissue section (reviewed, e.g., in Emmert-Buck, M.R. et al. (1996). Science 274, 998-1001; Espina, V. et al. (2007) Expert Rev. Mol. Diagn. 7, 647-657). The preparation of the cryostat sections and the capturing step using a laser capture microspope (Arcturus Veritas^{™} Laser Capture Microdissection Instrument (Molecular Devices, Inc., Sunnyvale, CA, USA) were performed essentially according to the instructions of the manufacturer.

The purification of total RNA from the samples was performed according to the guanidinium thiocyanate-phenol-chloroform extraction method (Chomczynski, P. and Sacchi, N. (1987) Anal. Biochem. 162, 156-159) using TRIzol reagent (Invitrogen Corporation, Carlsbad, CA, USA) according to the manufacturer's instructions.

The population of miRNAs was isolated via the *mir*Vana™ miRNA Isolation Kit (Ambion, Inc., Austin, TX, USA) according to the manufacturer's instruction.

### Example 2: Analysis of the miRNA expression profile in the samples

A qualitative analysis of the miRNAs (differentially) expressed in a particular sample may optionally be performed using the Agilent miRNA microarray platform (Agilent Technologies, Santa Clara, CA, USA) according to the manufacturer's instructions. The raw data obtained for single-color (CY3) hybridization were normalized by applying a Quantile method and using the R software known in the art.

Prior to miRNA expression analysis, a synthesized heterogenous miRNA may be added ("spiked-in") to the samples at certain ratio with respect to the total RNA concentration as an internal positive control for quantitative analysis. Such a "spike-in" miRNA may be a plant miRNA, for example, ath-miR168a, ath-miR162a, ppt-miR898b, or smo-mir1100, that has a low homology to human gene or transcript sequences. Alternatively, the "spike-in" miRNA may be any sequence 18 nt. to 30 nt. in length that is less than 70% homolog to human gene or transcript sequences.

For verifying (and/or quantifying) the miRNA expression data obtained an established real-time quantitative RT-PCR employing a TaqMan MicroRNA assay (Applied Biosystems, Foster City, CA, USA) was used according to the manufacturer's instructions.

For assessing whether a particular miRNA is differentially expressed in cancerogenous target cells as compared to control cells the following criteria were used:
(i) p-value (probability value) of ≤ 0.05 with an ≥ 2-fold change in differential expression in at least 30% of the tumor samples tested; and
(ii) p-value of ≤ 0.05/273 (the factor 273 is due to a Bonferroni correction, since 273 human miRNAs revealed a positive signal on the Agilent miRNA microarray).

In case, at least of these criteria was fulfilled, the miRNA was considered to be differentially expressed in the target and control cells, respectively.

Typically, at least three independent experiments were performed for each measurement and the miRNA expression level determined represents the mean value of the respective individual data obtained.

The expression data are summarized in Tables 4 to 6 below. Table 4 lists the miRNAs exhibiting a differential expression both in hepatocellular carcinoma (HCC) and carcinoma embolus (CE). Table 5 summarizes the miRNAs exhibiting a differential expression only in hepatocellular carcinoma, whereas Table 6 lists the miRNAs exhibiting a differential expression only in carcinoma embolus. In the column "ID", the abbreviation "k" denotes a known miRNA in hepatocellular cancer, whereas "n" denotes a newly identified miRNA. The expression levels and the degree of differential expression are listed in the columns "geometric mean". "SENS." denotes the sensitivity of detection, that is, the occurrence of the differentially expressed miRNA in HCC or CE samples. Particularly preferred miRNAs (SEQ ID NO: 1 to SEQ ID NO: 13 in Table 4; SEQ ID NO:37 to SEQ ID NO:47 in Table 5; and SEQ ID NO:71 to SEQ ID NO:79 in Table 6, respectively) are shown in bold.

**TABLE 4**

| **Hepatocellular carcinoma & carcinoma embolus** | | | | | | | | |
|---|---|---|---|---|---|---|---|---|
| | | | **T-TEST** | | **GEOMETRIC MEAN** | | | |
| **ID** | **hsa-miRNA** | **TUMOR** | **P-VAL.** | **Q-VAL.** | **NORMAL** | **TUMOR** | **FOLD** | **SENS.** |
| k | hsa-miR-100 | HCC | 0.026 | 7.08 | 268 | 121 | 0.5 | 56% |
| | | CE | 0.000 | 0.06 | 238 | 62 | 0.3 | 100% |
| k | hsa-miR-101 | HCC | 0.000 | 0.07 | 277 | 87 | 0.3 | 78% |
| | | CE | 0.002 | 0.42 | 286 | 68 | 0.2 | 100% |
| k | hsa-miR-10a | HCC | 0.024 | 6.57 | 105 | 56 | 0.5 | 33% |
| | | CE | 0.000 | 0.13 | 91 | 33 | 0.4 | 80% |
| k | hsa-miR-139-3p | HCC | 0.026 | 7.08 | 35 | 15 | 0.4 | 78% |
| | | CE | 0.016 | 4.33 | 32 | 4 | 0.1 | 100% |
| k | **hsa-miR-139-5p** | **HCC** | **0.030** | **8.16** | **46** | **9** | **0.2** | **67%** |
| | | **CE** | **0.004** | **0.97** | **40** | **4** | **0.1** | **80%** |
| k | hsa-miR-148a | HCC | 0.001 | 0.23 | 1617 | 718 | 0.4 | 56% |
| | | CE | 0.004 | 1.20 | 1662 | 451 | 0.3 | 100% |
| k | **hsa-miR-221** | **HCC** | **0.000** | **0.02** | **11** | **41** | **3.7** | **78%** |
| | | **CE** | **0.025** | **6.70** | **10** | **24** | **2.3** | **60%** |
| k | hsa-miR-30c | HCC | 0.020 | 5.58 | 391 | 243 | 0.6 | 56% |
| | | CE | 0.004 | 1.12 | 370 | 156 | 0.4 | 40% |
| k | **hsa-miR-324-5p** | **HCC** | **0.001** | **0.15** | **24** | **51** | **2.1** | **56%** |
| | | **CE** | **0.001** | **0.35** | **22** | **53** | **2.4** | **60%** |
| k | **hsa-miR-96** | **HCC** | **0.000** | **0.05** | **4** | **27** | **6.3** | **89%** |
| | | **CE** | **0.000** | **0.02** | **5** | **39** | **7.4** | **100%** |
| n | hsa-miR-122* | HCC | 0.005 | 1.34 | 1487 | 437 | 0.3 | 78% |
| | | CE | 0.000 | 0.01 | 1508 | 407 | 0.3 | 100% |
| n | **hsa-miR-144** | **HCC** | **0.000** | **0.01** | **44** | **6** | **0.1** | **100%** |
| | | **CE** | **0.001** | **0.25** | **43** | **7** | **0.2** | **100%** |
| n | **hsa-miR-144*** | **HCC** | **0.001** | **0.32** | **6** | **0** | **0.1** | **100%** |
| | | **CE** | **0.010** | **2.65** | **6** | **0** | **0.0** | **100%** |
| n | hsa-miR-152 | HCC | 0.010 | 2.80 | 20 | 9 | 0.5 | 89% |
| | | CE | 0.046 | 12.48 | 19 | 1 | 0.1 | 100% |
| n | **hsa-miR-18a** | **HCC** | **0.003** | **0.84** | **9** | **34** | **3.8** | **78%** |
| | | **CE** | **0.023** | **6.20** | **10** | **26** | **2.6** | **60%** |
| n | **hsa-miR-18b** | **HCC** | **0.006** | **1.50** | **5** | **14** | **3.0** | **67%** |
| | | **CE** | **0.042** | **11.42** | **5** | **10** | **2.0** | **40%** |
| n | hsa-miR-24-1* | HCC | 0.019 | 5.23 | 14 | 3 | 0.2 | 78% |
| | | CE | 0.001 | 0.24 | 13 | 3 | 0.3 | 100% |
| n | hsa-miR-26b | HCC | 0.018 | 4.82 | 980 | 520 | 0.5 | 67% |
| | | CE | 0.000 | 0.07 | 963 | 408 | 0.4 | 80% |
| n | hsa-miR-29c* | HCC | 0.043 | 11.81 | 20 | 12 | 0.6 | 67% |
| | | CE | 0.006 | 1.77 | 18 | 5 | 0.3 | 80% |
| n | **hsa-miR-30d** | **HCC** | **0.000** | **0.01** | **267** | **638** | **2.4** | **67%** |
| | | **CE** | **0.009** | **2.43** | **251** | **711** | **2.8** | **60%** |
| n | hsa-miR-30e | HCC | 0.007 | 1.78 | 287 | 165 | 0.6 | 67% |
| | | CE | 0.019 | 5.23 | 290 | 134 | 0.5 | 60% |
| n | hsa-miR-30e* | HCC | 0.015 | 4.08 | 64 | 27 | 0.4 | 78% |
| | | CE | 0.001 | 0.36 | 57 | 9 | 0.2 | 100% |
| n | hsa-miR-378 | HCC | 0.010 | 2.81 | 73 | 26 | 0.4 | 67% |
| | | CE | 0.013 | 3.44 | 69 | 6 | 0.1 | 80% |
| n | hsa-miR-378* | HCC | 0.006 | 1.71 | 26 | 8 | 0.3 | 67% |
| | | CE | 0.007 | 1.88 | 26 | 2 | 0.1 | 80% |
| n | hsa-miR-424 | HCC | 0.000 | 0.02 | 284 | 107 | 0.4 | 78% |
| | | CE | 0.001 | 0.27 | 276 | 101 | 0.4 | 80% |
| n | **hsa-miR-450a** | **HCC** | **0.017** | **4.55** | **9** | **1** | **0.2** | **100%** |
| | | **CE** | **0.003** | **0.77** | **9** | **1** | **0.1** | **100%** |
| n | hsa-miR-455-3p | HCC | 0.007 | 1.89 | 184 | 96 | 0.5 | 56% |
| | | CE | 0.001 | 0.39 | 173 | 61 | 0.4 | 80% |
| n | hsa-miR-455-5p | HCC | 0.003 | 0.77 | 21 | 8 | 0.4 | 56% |
| | | CE | 0.003 | 0.93 | 23 | 5 | 0.2 | 100% |
| n | hsa-miR-505 | HCC | 0.003 | 0.78 | 64 | 25 | 0.4 | 56% |
| | | CE | 0.001 | 0.15 | 58 | 8 | 0.1 | 100% |
| n | hsa-miR-542-3p | HCC | 0.000 | 0.07 | 9 | 3 | 0.3 | 78% |
| | | CE | 0.036 | 9.93 | 9 | 1 | 0.1 | 100% |
| n | hsa-miR-99a | HCC | 0.011 | 3.00 | 468 | 224 | 0.5 | 56% |
| | | CE | 0.000 | 0.01 | 440 | 92 | 0.2 | 100% |
| n | hsa-miR-29c | HCC | 0.004 | 1.15 | 833 | 481 | 0.6 | 33% |
| | | CE | 0.027 | 7.50 | 803 | 472 | 0.6 | 40% |
| n | **hsa-miR-331-3p** | **HCC** | **0.001** | **0.31** | **103** | **250** | **2.4** | **78%** |
| | | **CE** | **0.004** | **1.16** | **100** | **301** | **3.0** | **60%** |
| n | hsa-miR-338-3p | HCC | 0.001 | 0.19 | 46 | 22 | 0.5 | 67% |
| | | CE | 0.000 | 0.02 | 47 | 11 | 0.2 | 100% |
| n | **hsa-miR-183** | **HCC** | **0.001** | **0.32** | **1** | **11** | **15.6** | **89%** |
| | | **CE** | **0.019** | **5.16** | **1** | **12** | **22.1** | **100%** |
| n | **hsa-miR-551b** | **HCC** | **0.007** | **2.00** | **2** | **28** | **16.3** | **100%** |
| | | **CE** | **0.048** | **13.17** | **1** | **23** | **21.8** | **100%** |

**TABLE 5**

| **Hepatocellular carcinoma** | | | | | | | | |
|---|---|---|---|---|---|---|---|---|
| | | | **T-TEST** | | **GEOMETRIC MEAN** | | | |
| **ID** | **hsa-miRNA** | **TUMOR** | **P-VAL.** | **Q-VAL.** | **NORMAL** | **TUMOR** | **FOLD** | **SENS.** |
| k | hsa-miR-130b | HCC | 0.014 | 3.76 | 25 | 62 | 2.51 | 67% |
| k | hsa-miR-132 | HCC | 0.033 | 8.89 | 6 | 10 | 1.69 | 44% |
| k | hsa-miR-194 | HCC | 0.000 | 0.12 | 912 | 537 | 0.59 | 33% |
| k | hsa-miR-199b-3p | HCC | 0.043 | 11.86 | 607 | 393 | 0.65 | 33% |
| k | hsa-miR-215 | HCC | 0.003 | 0.73 | 675 | 380 | 0.56 | 44% |
| k | **hsa-miR-224** | **HCC** | **0.019** | **5.10** | **24** | **90** | **3.72** | **67%** |
| n | hsa-miR-106b | HCC | 0.028 | 7.60 | 142 | 248 | 1.75 | 44% |
| n | hsa-miR-125a-5p | HCC | 0.046 | 12.53 | 45 | 89 | 2.01 | 44% |
| n | hsa-miR-151-3p | HCC | 0.005 | 1.37 | 27 | 58 | 2.14 | 56% |
| n | hsa-miR-17 | HCC | 0.028 | 7.75 | 194 | 395 | 2.04 | 33% |
| n | hsa-miR-21* | HCC | 0.027 | 7.41 | 10 | 23 | 2.24 | 44% |
| n | hsa-miR-25 | HCC | 0.017 | 4.61 | 134 | 272 | 2.03 | 44% |
| n | hsa-miR-301a | HCC | 0.031 | 8.37 | 9 | 21 | 2.35 | 56% |
| n | **hsa-miR-375** | **HCC** | **0.032** | **8.73** | **46** | **13** | **0.28** | **67%** |
| n | hsa-miR-423-5p | HCC | 0.011 | 3.07 | 17 | 28 | 1.64 | 44% |
| n | hsa-miR-425 | HCC | 0.003 | 0.85 | 42 | 80 | 1.93 | 56% |
| n | **hsa-miR-451** | **HCC** | **0.000** | **0.09** | **1919** | **628** | **0.33** | **78%** |
| n | hsa-miR-484 | HCC | 0.020 | 5.59 | 12 | 20 | 1.70 | 56% |
| n | hsa-miR-500* | HCC | 0.001 | 0.38 | 5 | 15 | 2.98 | 67% |
| n | hsa-miR-502-3p | HCC | 0.013 | 3.63 | 6 | 13 | 2.42 | 67% |
| n | hsa-miR-502-5p | HCC | 0.028 | 7.66 | 2 | 5 | 2.49 | 56% |
| n | **hsa-miR-532-3p** | **HCC** | **0.000** | **0.02** | **6** | **24** | **3.91** | **100%** |
| n | hsa-miR-532-5p | HCC | 0.001 | 0.41 | 13 | 34 | 2.63 | 67% |
| n | **hsa-miR-663** | **HCC** | **0.030** | **8.26** | **12** | **40** | **3.23** | **67%** |
| n | hsa-miR-769-5p | HCC | 0.007 | 2.01 | 2 | 5 | 2.83 | 67% |
| n | hsa-miR-93 | HCC | 0.005 | 1.32 | 80 | 177 | 2.21 | 67% |
| n | **hsa-miR-99b** | **HCC** | **0.000** | **0.08** | **16** | **49** | **3.13** | **89%** |
| n | hsa-miR-181b | HCC | 0.010 | 2.74 | 19 | 40 | 2.06 | 44% |
| n | **hsa-miR-335** | **HCC** | **0.019** | **5.13** | **18** | **5** | **0.27** | **78%** |
| n | **hsa-miR-362-5p** | **HCC** | **0.000** | **0.11** | **8** | **29** | **3.41** | **78%** |
| n | hsa-miR-501-5p | HCC | 0.014 | 3.94 | 8 | 13 | 1.60 | 33% |
| n | **hsa-miR-652** | **HCC** | **0.011** | **2.97** | **1** | **10** | **7.86** | **100%** |
| n | **hsa-miR-222** | **HCC** | **0.004** | **0.99** | **1** | **12** | **14.11** | **100%** |
| n | **hsa-miR-501-3p** | **HCC** | **0.002** | **0.46** | **0** | **5** | **10.88** | **100%** |

**TABLE 6**

| **Carcinoma embolus** | | | | | | | | |
|---|---|---|---|---|---|---|---|---|
| | | | **T-TEST** | | **GEOMETRIC MEAN** | | | |
| **ID** | **hsa-miRNA** | **TUMOR** | **P-VAL.** | **Q-VAL.** | **NORMAL** | **TUMOR** | **FOLD** | **SENS.** |
| k | hsa-let-7c | CE | 0.0249 | 6.81 | 620 | 317 | 0.51 | 40% |
| k | hsa-miR-122 | CE | 0.0000 | 0.00 | 16552 | 5349 | 0.32 | 100% |
| k | hsa-miR-125b | CE | 0.0221 | 6.03 | 1083 | 543 | 0.50 | 80% |
| k | hsa-miR-130a | CE | 0.0122 | 3.33 | 282 | 111 | 0.39 | 60% |
| k | hsa-miR-195 | CE | 0.0034 | 0.92 | 275 | 124 | 0.45 | 80% |
| k | **hsa-miR-199b-5p** | **CE** | **0.0289** | **7.89** | **5** | **12** | **2.25** | **60%** |
| k | **hsa-miR-21** | **CE** | **0.0000** | **0.00** | **5427** | **16552** | **3.05** | **100%** |
| k | hsa-miR-30a | CE | 0.0071 | 1.93 | 266 | 101 | 0.38 | 60% |
| n | **hsa-miR-125b-2*** | **CE** | **0.0111** | **3.02** | **10** | **0** | **0.03** | **100%** |
| n | hsa-miR-126* | CE | 0.0102 | 2.80 | 23 | 7 | 0.31 | 80% |
| n | hsa-miR-128 | CE | 0.0150 | 4.08 | 21 | 9 | 0.44 | 60% |
| n | hsa-miR-192* | CE | 0.0452 | 12.34 | 29 | 10 | 0.35 | 60% |
| n | hsa-miR-28-5p | CE | 0.0496 | 13.54 | 107 | 48 | 0.45 | 60% |
| n | hsa-miR-30a* | CE | 0.0002 | 0.05 | 35 | 5 | 0.14 | 100% |
| n | hsa-miR-30b* | CE | 0.0141 | 3.84 | 7 | 2 | 0.35 | 60% |
| n | **hsa-miR-30c-2*** | **CE** | **0.0153** | **4.17** | **4** | **0** | **0.07** | **100%** |
| n | hsa-miR-486-5p | CE | 0.0039 | 1.08 | 14 | 5 | 0.32 | 80% |
| n | hsa-miR-497 | CE | 0.0025 | 0.70 | 120 | 44 | 0.36 | 80% |
| n | **hsa-miR-505*** | **CE** | **0.0462** | **12.61** | **8** | **1** | **0.08** | **100%** |
| n | hsa-miR-542-5p | CE | 0.0026 | 0.71 | 6 | 2 | 0.43 | 80% |
| n | hsa-miR-564 | CE | 0.0450 | 12.27 | 5 | 3 | 0.51 | 60% |
| n | hsa-miR-590-5p | CE | 0.0421 | 11.49 | 30 | 12 | 0.40 | 60% |
| n | **hsa-miR-19b** | **CE** | **0.0397** | **10.83** | **712** | **1227** | **1.72** | **40%** |
| n | **hsa-miR-203** | **CE** | **0.0374** | **10.22** | **7** | **0.13** | **0.02** | **0.40** |
| n | hsa-miR-340 | CE | 0.0038 | 1.05 | 27 | 8 | 0.29 | 100% |
| n | hsa-miR-582-5p | CE | 0.0373 | 10.17 | 10 | 6 | 0.60 | 40% |
| n | hsa-miR-194* | CE | 0.0193 | 5.28 | 6 | 3 | 0.42 | 40% |
| n | **hsa-miR-103** | **CE** | **0.0489** | **13.35** | **397** | **640** | **1.61** | **60%** |
| n | **hsa-miR-23a** | **CE** | **0.0397** | **10.85** | **505** | **867** | **1.72** | **60%** |

The results obtained demonstrate a global highly specific regulation of miRNA expression in hepatocellular cancer. Thus, the respective subsets of miRNAs specified herein represent unique miRNA expression signatures for expression profiling of hepatocellular cancer that do not only allow the identification of a cancerogenous state as such but also enables the discrimination between different types of tumors, namely hepatocellular carcinoma (a primary malignancy) and carcinoma embolus (a secondary malignancy).

Hence, the miRNA expression signatures defined herein do not run out in the mere identification of hepatocellular cancer (HCC) but also enable a reliably risk assessment of patients exhibiting or being supposed to have a pre-cancerous state (e.g., a hepatic cirrhosis) whether or not the pre-cancerous state will progress in a carcinoma. In other words, the miRNA expression signatures as defined herein enable a prediction of disease progression for patients having a predisposition to develop HCC.

Such a risk assessment of cancer progression is of significant clinical importance in several respects. The identification of the miRNA expression signatures of the present invention provides a unique molecular marker that allows the detection of HCC at an early disease stage (that is, at a stage where the presence of malignant cells is not yet detectable by *in situ* techniques or microscopic analysis of biopsy or resection material), where HCC still can be efficiently treated markedly. Furthermore, the prediction of cancer progression may be used to guide the therapy decision in patients exhibiting a pre-cancerous state of HCC.

The present invention illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by embodiments and optional features, modifications and variations of the inventions embodied therein may be resorted to by those skilled in the art, and that such modifications and variations are considered to be within the scope of this invention.

The invention has been described broadly and generically herein. Each of the narrower species and sub-generic groupings falling within the generic disclosure also form part of the invention. This includes the generic description of the invention with a proviso or negative limitation removing any subject matter from the genus, regardless of whether or not the excised material is specifically recited herein.

Other embodiments are within the following claims. In addition, where features or aspects of the invention are described in terms of Markush groups, those skilled in the art will recognize that the invention is also thereby described in terms of any individual member or subgroup of members of the Markush group.

## Claims

1. Diagnostic kit of molecular markers for identifying one or more mammalian target cells exhibiting or having a predisposition to develop hepatocellular cancer, the kit comprising a plurality of nucleic acid molecules, each nucleic acid molecule encoding a microRNA sequence,
wherein one or more of the plurality of nucleic acid molecules are differentially expressed in the target cells and in one or more control cells, and
wherein the one or more differentially expressed nucleic acid molecules together represent a nucleic acid expression signature that is indicative for the presence of or the predisposition to develop hepatocellular cancer.

2. The kit of claim 1, wherein the nucleic acid expression signature comprises at least five nucleic acid molecules, preferably at least eight nucleic acid molecules, and particularly preferably at least twelve nucleic acid molecules.

3. The kit of claim 1 or 2, wherein the nucleic acid expression signature comprises at least one nucleic acid molecule encoding a microRNA sequence whose expression is up-regulated in the one or more target cells compared to the one or more control cells and at least one nucleic acid molecule encoding a microRNA sequence whose expression is down-regulated in the one or more target cells compared to the one or more control cells.

4. The kit of any of claims 1 to 3, wherein the nucleic acid expression signature comprises any one or more of the nucleic acid molecules encoding hsa-mir-221, hsa-mir-324-5p, hsa-mir-96, hsa-mir-18a, hsa-mir-18b, hsa-mir-30d, hsa-mir-331-3p, hsa-mir-183, hsa-mir-551b, hsa-mir-139-5p, hsa-mir-144, hsa-mir-144*, and hsa-mir-450a.

5. The kit of claim 4, wherein the expression of any one or more of the nucleic acid molecules encoding hsa-mir-221, hsa-mir-324-5p, hsa-mir-96, hsa-mir-18a, hsa-mir-18b, hsa-mir-30d, hsa-mir-331-3p, hsa-mir-183, and hsa-mir-551b is up-regulated and the expression of any one or more of the nucleic acid molecules hsa-mir-139-5p, hsa-mir-144, hsa-mir-144*, and hsa-mir-450a is down-regulated in the in the one or more target cells compared to the one or more control cells.

6. The kit of any of claims 1 to 5, for the further use of discriminating hepatocellular cancer selected from the group consisting of hepatocellular carcinoma and carcinoma embolus.

7. The kit of claim 6, wherein the hepatocellular cancer is hepatocellular carcinoma, and
wherein the nucleic acid expression signature comprises any one or more of the nucleic acid molecules encoding hsa-miR-224, hsa-miR-532-3p, hsa-miR-663, hsa-miR-99b, hsa-miR-362-5p, hsa-miR-652, hsa-miR-222, hsa-miR-501-3p, hsa-miR-375, hsa-miR-451, and hsa-miR-335

8. The kit of claim 7, wherein the expression of any one or more of the nucleic acid molecules encoding hsa-miR-224, hsa-miR-532-3p, hsa-miR-663, hsa-miR-99b, hsa-miR-362-5p, hsa-miR-652, hsa-miR-222, and hsa-miR-501-3p is up-regulated and the expression of any one or more of the nucleic acid molecules hsa-miR-375, hsa-miR-451, and hsa-miR-335 is down-regulated in the in the one or more target cells compared to the one or more control cells.

9. The kit of claim 6, wherein the hepatocellular cancer is carcinoma embolus, and
wherein the nucleic acid expression signature comprises any one or more of the nucleic acid molecules encoding hsa-miR-199b-5p, hsa-miR-21, hsa-miR-19b, hsa-miR-103, hsa-miR-23a, hsa-miR-125b-2*, hsa-miR-30c-2*, hsa-miR-505*, and hsa-miR-203.

10. The kit of claim 9, wherein the expression of any one or more of the nucleic acid molecules encoding hsa-miR-199b-5p, hsa-miR-21, hsa-miR-19b, hsa-miR-103, and hsa-miR-23a is up-regulated and the expression of any one or more of the nucleic acid molecules hsa-miR-125b-2*, hsa-miR-30c-2*, hsa-miR-505*, and hsa-miR-203 is down-regulated in the in the one or more target cells compared to the one or more control cells.

11. Method for identifying one or more mammalian target cells exhibiting or having a predisposition to develop hepatocellular cancer, the method comprising:
(a) determining in the one or more target cells the expression levels of a plurality of nucleic acid molecules, each nucleic acid molecule encoding a microRNA sequence;
(b) determining the expression levels of the plurality of nucleic acid molecules in one or more control cells; and
(c) identifying from the plurality of nucleic acid molecules one or more nucleic acid molecules that are differentially expressed in the target and control cells by comparing the respective expression levels obtained in steps (a) and (b),
wherein the one or more differentially expressed nucleic acid molecules together represent a nucleic acid expression signature, as defined in any of claims 1 to 10, that is indicative for the presence of or the predisposition to develop hepatocellular cancer.

12. The method of claim 11, for the further use of discriminating hepatocellular cancer selected from the group consisting of hepatocellular carcinoma and carcinoma embolus.

13. Method for preventing or treating hepatocellular cancer in one or more mammalian target cells, the method comprising:
(a) identifying in one or more target cells a nucleic acid expression signature by using a method as defined in claim 11 or 12; and
(b) modifying in the one or more cells the expression of one or more nucleic acid molecules encoding a microRNA sequence that is/are comprised in the nucleic acid expression signature in such way that the expression of a nucleic acid molecule whose expression is up-regulated in the one or more target cells is down-regulated and the expression of a nucleic acid molecule whose expression is down-regulated in the one or more target cells is up-regulated.

14. Pharmaceutical composition for the prevention and/or treatment of hepatocellular cancer in one or more mammalian target cells, the composition comprising one or more nucleic acid molecules, each nucleic acid molecule encoding a sequence that is at least partially complementary to a microRNA sequence encoded by a nucleic acid molecule whose expression is up-regulated in the one or more target cells, as defined in any of claims 1 to 10, and/or that corresponds to a microRNA sequence encoded by a nucleic acid molecule whose expression is down-regulated in the one or more target cells, as defined in any of claims 1 to 10.

15. Use of the pharmaceutical composition of claim 14 for the manufacture of a medicament for the prevention and/or treatment of hepatocellular cancer.
